# EUROPEAN PATENT APPLICATION

(11) **EP 3 950 764 A1**
(43) Date of publication of application: **09.02.2022**
(21) Application number: 20785387.0
(22) Date of filing: 16.03.2020
(51) Int. Cl.: C08G 61/00, C09K 11/06, H01L 51/50

(54) **LIGHT-EMITTING ELEMENT AND COMPOSITION FOR LIGHT-EMITTING ELEMENT**

(30) Priority: 29.03.2019 JP 2019066177
(71) Applicant: SUMITOMO CHEMICAL COMPANY LIMITED, Chuo-ku Tokyo 104-8260 (JP)
(72) Inventor: SASADA, Toshiaki, Tsukuba-shi, Ibaraki 300-3294 (JP); MATSUMOTO, Ryuji, Ishikawa 923-1201 (JP)
(74) Representative: J A Kemp LLP
(86) International application number: PCT/JP2020/011383
(87) International publication number: WO 2020/203209

(57) **Abstract**

To provide a composition which is useful for producing a light emitting device excellent in light emission efficiency, and a light emitting device comprising the composition.

A light emitting device comprising an anode, a cathode, and an organic layer disposed between the anode and the cathode and containing a composition for light emitting device, wherein the composition for light emitting device contains a metal complex represented by the formula (2), and a compound (B) having a condensed hetero ring skeleton (b) containing a boron atom and at least one selected from the group consisting of an oxygen atom, a sulfur atom, a selenium atom, an sp³ carbon atom and a nitrogen atom in the ring.

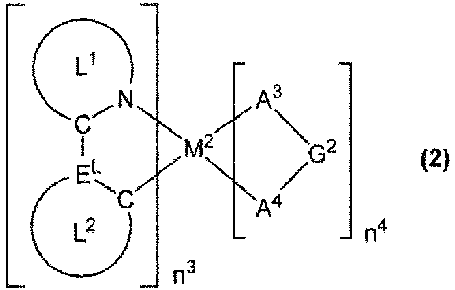

**-R^{1T} (1-T)**

## Description

### Technical Field

The present invention relates to a light emitting device and a composition for light emitting device.

### Background Art

Light emitting devices such as an organic electroluminescent device and the like can be suitably used, for example, for display and illumination. As the light emitting material used for a light emitting layer of a light emitting device, for example, Patent Document 1 suggests a composition containing a compound B0, and a metal complex G1 or a metal complex Firpic.

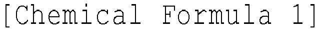

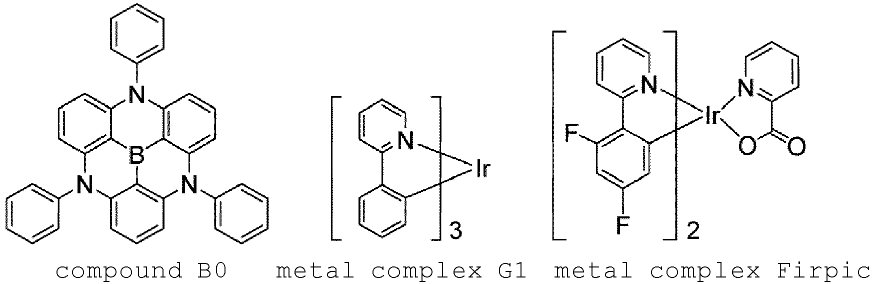

Further, as the light emitting material used for a light emitting layer of a light emitting device, for example, Patent Document 2 suggests a composition containing a thermally activated delayed fluorescent (TADF) compound T0 and a metal complex G3.

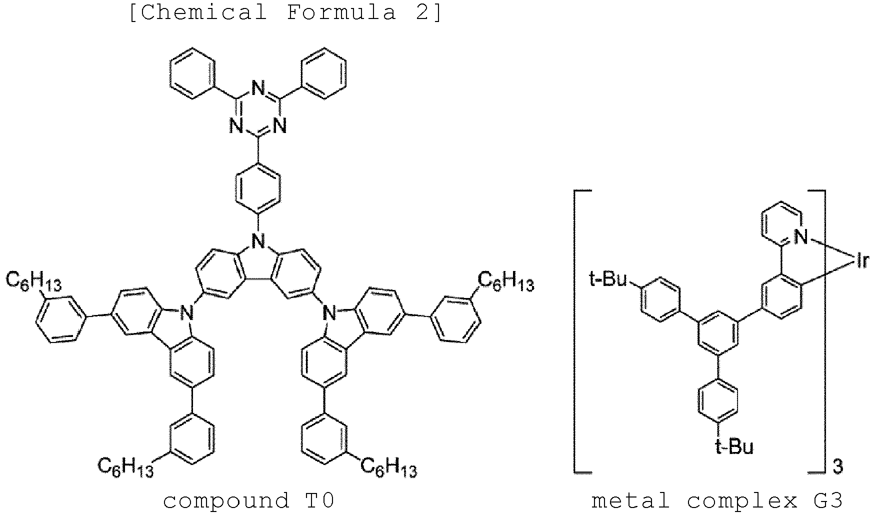

### [Prior Art Document]

### [Patent Document]

[Patent Document 1] Japanese Unexamined Patent Application Publication (JP-A) No. 2018-043984
[Patent Document 2] International Publication WO2017/154884

### Summary of the Invention

### Problem to be Solved by the Invention

However, light emitting devices fabricated using the above-described compositions were not necessarily sufficient in light emission efficiency.

Then, the present invention has an object of providing a composition which is useful for producing a light emitting device excellent in light emission efficiency, and a light emitting device comprising the composition.

### Means for Solving the Problem

The present inventors have intensively studied to solve the above-described problem and resultantly found that it becomes possible to form a light emitting device excellent in light emission efficiency by combination of a specific metal complex and a specific compound (B), leading to completion of the present invention. The metal complex G1 in Patent Document 1 is a metal complex not having a group represented by the formula (1-T) described later. Further, the compound T0 in Patent Document 2 is a compound not having a condensed hetero ring skeleton (b) described later.

That is, the present invention provides the following [1] to [20].
[1] A light emitting device comprising
   an anode,
   a cathode, and
   an organic layer disposed between the above-described anode and the above-described cathode and containing a composition for light emitting device, wherein
   the above-described composition for light emitting device contains
      a metal complex represented by the formula (2) and
      a compound (B) having a condensed hetero ring skeleton (b) containing a boron atom and at least one selected from the group consisting of an oxygen atom, a sulfur atom, a selenium atom, an sp³ carbon atom and a nitrogen atom in the ring: 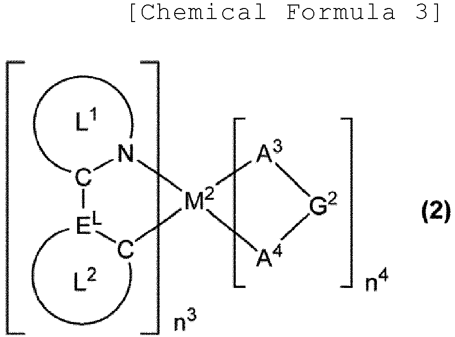 [wherein,
         M² represents a rhodium atom, a palladium atom, an iridium atom or a platinum atom.
         n³ represents an integer of 1 or more, and n⁴ represents an integer of 0 or more. n³+n⁴ is 3 when M² is a rhodium atom or an iridium atom, while n³+n⁴ is 2 when M² is a palladium atom or a platinum atom.
         E^{L} represents a carbon atom or a nitrogen atom. When a plurality of E^{L} are present, they may be the same or different.
         Ring L¹ represents an aromatic hetero ring containing a 6-membered ring, and this ring optionally has a substituent. When a plurality of the substituents are present, they may be the same or different and may be combined together to form a ring together with atoms to which they are attached. When a plurality of Ring L¹ are present, they may be the same or different.
         Ring L² represents an aromatic hydrocarbon ring or an aromatic hetero ring, and these rings optionally have a substituent. When a plurality of the substituents are present, they may be the same or different and may be combined together to form a ring together with atoms to which they are attached. When a plurality of Ring L² are present, they may be the same or different.

The substituent which Ring L¹ optionally has and the substituent which Ring L² optionally has may be the same or different and may be combined together to form a ring together with atoms to which they are attached.

At least one of Ring L¹ and Ring L² has a group represented by the formula (1-T) as the substituent. When a plurality of the groups represented by the formula (1-T) are present, they may be the same or different.

A³-G²-A⁴ represents an anionic bidentate ligand. A³ and A⁴ each independently represent a carbon atom, an oxygen atom or a nitrogen atom, and these atoms may be ring-constituent atoms. G² represents a single bond, or an atomic group constituting a bidentate ligand together with A³ and A⁴. When a plurality of A³-G²-A⁴ are present, they may be the same or different.]

[Chemical Formula 4] **-R^{1T} (1-T)**

[wherein, R^{1T} represents an alkyl group, a cycloalkyl group, an alkoxy group, a cycloalkoxy group, an aryloxy group, an aryl group, a monovalent hetero ring group or a substituted amino group, and these groups optionally have a substituent. When a plurality of the substituents are present, they may be the same or different and may be combined together to form a ring together with atoms to which they are attached.].

[2] The light emitting device according to [1], wherein the above-described Ring L¹ is a pyridine ring, a diazabenzene ring, an azanaphthalene ring or a diazanaphthalene ring, and these rings optionally have a substituent.

[3] The light emitting device according to [2], wherein
the above-described Ring L¹ is a pyridine ring, a diazabenzene ring, a quinoline ring or a diazanaphthalene ring, and these rings optionally have a substituent, and
the above-described R^{1T} is an alkyl group, a cycloalkyl group, an aryl group, a monovalent hetero ring group or a substituted amino group, and these groups optionally have a substituent.

[4] The light emitting device according to [2], wherein
the above-described Ring L¹ is an isoquinoline ring optionally having a substituent, and
the above-described R^{1T} is an aryl group, a monovalent hetero ring group or a substituted amino group, and these groups optionally have a substituent.

[5] The light emitting device according to any one of [1] to [4], wherein
the above-described Ring L² is a benzene ring, a pyridine ring or a diazabenzene ring, and these rings optionally have a substituent.

[6] The light emitting device according to any one of [1] to [5], wherein
the above-described R^{1T} is an aryl group optionally having a substituent or a monovalent hetero ring group optionally having a substituent.

[7] The light emitting device according to any one of [1] to [6], wherein
the above-described condensed hetero ring skeleton (b) contains a boron atom and at least one selected from the group consisting of an oxygen atom, a sulfur atom and a nitrogen atom in the ring.

[8] The light emitting device according to [7], wherein
the above-described condensed hetero ring skeleton (b) contains a boron atom and a nitrogen atom in the ring.

[9] The light emitting device according to any one of [1] to [6], wherein
the above-described compound (B) is a compound represented by the formula (1-1), a compound represented by the formula (1-2) or a compound represented by the formula (1-3): 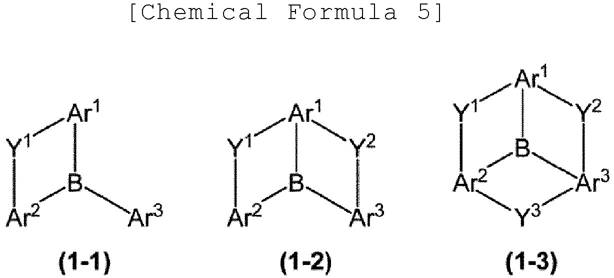 [wherein,
Ar¹, Ar² and Ar³ each independently represent an aromatic hydrocarbon group or a hetero ring group, and these groups optionally have a substituent. When a plurality of the substituents are present, they may be the same or different and may be combined together to form a ring together with atoms to which they are attached.
Y¹ represents an oxygen atom, a sulfur atom, a selenium atom, a group represented by -N(Ry)-, an alkylene group or a cycloalkylene group, and these groups optionally have a substituent. When a plurality of the substituents are present, they may be the same or different and may be combined together to form a ring together with atoms to which they are attached.
Y² and Y³ each independently represent a single bond, an oxygen atom, a sulfur atom, a selenium atom, a group represented by -N(Ry)-, an alkylene group or a cycloalkylene group, and these groups optionally have a substituent. When a plurality of the substituents are present, they may be the same or different and may be combined together to form a ring together with atoms to which they are attached. Ry represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of Ry are present, they may be the same or different. Ry may be bonded directly or via a connecting group to Ar¹, Ar² or Ar³.].

[10] The light emitting device according to [9], wherein
the above-described Y¹, the above-described Y² and the above-described Y³ are each an oxygen atom, a sulfur atom or a group represented by -N(Ry)-.

[11] The light emitting device according to [10], wherein
the above-described Y¹, the above-described Y² and the above-described Y³ are each a group represented by -N(Ry)-.

[12] The light emitting device according to any one of [1] to [11], wherein
the absolute value of a different between the energy level of the lowest triplet excited state of the above-described compound (B) and the energy level of the lowest singlet excited state of the above-described compound (B) is 0.50 eV or less.

[13] The light emitting device according to any one of [1] to [12], wherein
the above-described composition for light emitting device further contains a compound represented by the formula (H-1): 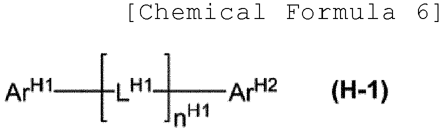 [wherein,
Ar^{H1} and Ar^{H2} each independently represent an aryl group, a monovalent hetero ring group or a substituted amino group, and these groups optionally have a substituent. When a plurality of the substituents are present, they may be the same or different and may be combined together to form a ring together with atoms to which they are attached.
n^{H1} represents an integer of 0 or more.
L^{H1} represents an arylene group, a divalent hetero ring group, an alkylene group or a cycloalkylene group, and these groups optionally have a substituent. When a plurality of the substituents are present, they may be the same or different and may be combined together to form a ring together with atoms to which they are attached. When a plurality of L^{H1} are present, they may be the same or different.].

[14] The light emitting device according to any one of [1] to [13], wherein
the above-described composition for light emitting device further contains at least one selected from the group consisting of a hole transporting material, a hole injection material, an electron transporting material, an electron injection material, a light emitting material, an antioxidant and a solvent.

[15] A composition for light emitting device comprising
a metal complex represented by the formula (2), and
a compound (B) having a condensed hetero ring skeleton (b) containing a boron atom and at least one selected from the group consisting of an oxygen atom, a sulfur atom, a selenium atom, an sp³ carbon atom and a nitrogen atom in the ring: 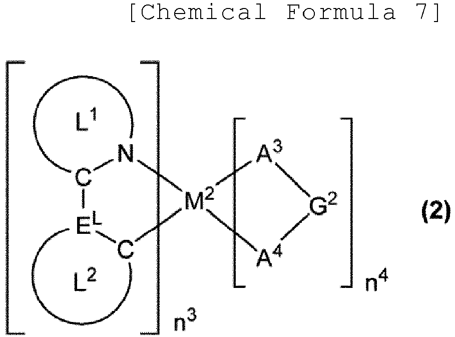 [wherein,
   M² represents a rhodium atom, a palladium atom, an iridium atom or a platinum atom.
   n³ represents an integer of 1 or more, and n⁴ represents an integer of 0 or more. n³+n⁴ is 3 when M² is a rhodium atom or an iridium atom, while n³+n⁴ is 2 when M² is a palladium atom or a platinum atom.
   E^{L} represents a carbon atom or a nitrogen atom. When a plurality of E^{L} are present, they may be the same or different.
   Ring L¹ represents an aromatic hetero ring containing a 6-membered ring, and this ring optionally has a substituent. When a plurality of the substituents are present, they may be the same or different and may be combined together to form a ring together with atoms to which they are attached. When a plurality of Ring L¹ are present, they may be the same or different.
   Ring L² represents an aromatic hydrocarbon ring or an aromatic hetero ring, and these rings optionally have a substituent. When a plurality of the substituents are present, they may be the same or different and may be combined together to form a ring together with atoms to which they are attached. When a plurality of Ring L² are present, they may be the same or different.

The substituent which Ring L¹ optionally has and the substituent which Ring L² optionally has may be the same or different and may be combined together to form a ring together with atoms to which they are attached.

At least one of Ring L¹ and Ring L² has a group represented by the formula (1-T) as the substituent. When a plurality of the groups represented by the formula (1-T) are present, they may be the same or different.

A³-G²-A⁴ represents an anionic bidentate ligand. A³ and A⁴ each independently represent a carbon atom, an oxygen atom or a nitrogen atom, and these atoms may be ring-constituent atoms. G² represents a single bond, or an atomic group constituting a bidentate ligand together with A³ and A⁴. When a plurality of A³-G²-A⁴ are present, they may be the same or different.]

[Chemical Formula 8] **-R^{1T} (1-T)**

[wherein, R^{1T} represents an alkyl group, a cycloalkyl group, an alkoxy group, a cycloalkoxy group, an aryloxy group, an aryl group, a monovalent hetero ring group or a substituted amino group, and these groups optionally have a substituent. When a plurality of the substituents are present, they may be the same or different and may be combined together to form a ring together with atoms to which they are attached.].

[16] The composition for light emitting device according to [15], wherein
the above-described Ring L¹ is a pyridine ring, a diazabenzene ring, a quinoline ring or a diazanaphthalene ring, and these rings optionally have a substituent, and
the above-described R^{1T} is an alkyl group, a cycloalkyl group, an aryl group, a monovalent hetero ring group or a substituted amino group, and these groups optionally have a substituent.

[17] The composition for light emitting device according to [15], wherein
the above-described Ring L¹ is an isoquinoline ring optionally having a substituent, and the above-described R^{1T} is an aryl group, a monovalent hetero ring group or a substituted amino group, and these groups optionally have a substituent.

[18] The composition for light emitting device according to any one of [15] to [17], wherein
the above-described condensed hetero ring skeleton (b) contains a boron atom and a nitrogen atom in the ring.

[19] The composition for light emitting device according to any one of [15] to [18], further comprising a compound represented by the formula (H-1): 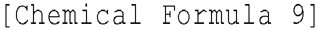 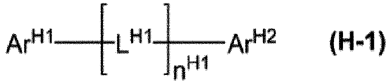 [wherein,
Ar^{H1} and Ar^{H2} each independently represent an aryl group, a monovalent hetero ring group or a substituted amino group, and these groups optionally have a substituent. When a plurality of the substituents are present, they may be the same or different and may be combined together to form a ring together with atoms to which they are attached.
n^{H1} represents an integer of 0 or more.
L^{H1} represents an arylene group, a divalent hetero ring group, an alkylene group or a cycloalkylene group, and these groups optionally have a substituent. When a plurality of the substituents are present, they may be the same or different and may be combined together to form a ring together with atoms to which they are attached. When a plurality of L^{H1} are present, they may be the same or different.].

[20] The composition for light emitting device according to any one of [15] to [19], further comprising at least one selected from the group consisting of a hole transporting material, a hole injection material, an electron transporting material, an electron injection material, a light emitting material, an antioxidant and a solvent.

### Effect of the Invention

According to the present invention, it is possible to provide a composition which is useful for producing a light emitting device excellent in light emission efficiency. Further, according to the present invention, it is possible to provide a light emitting device comprising the composition.

### Modes for Carrying out the Invention

Suitable embodiments of the present invention will be illustrated in detail below.

### <Explanation of common terms>

Terms commonly used in the present specification have the following meanings unless otherwise stated.

"Room temperature" denotes 25°C.

Me represents a methyl group, Et represents an ethyl group, Bu represents a butyl group, i-Pr represents an isopropyl group, and t-Bu represents a tert-butyl group.

A hydrogen atom may be a heavy hydrogen atom or a light hydrogen atom.

"The low molecular weight compound" means a compound having no molecular weight distribution and having a molecular weight of 1×10⁴ or less.

"The polymer compound" means a polymer having molecular weight distribution and having a polystyrene-equivalent number-average molecular weight of 1×10³ or more (for example, 1×10³ to 1×10⁸).

"The constitutional unit" means a unit occurring once or more times in the polymer compound.

The polymer compound may be any of a block copolymer, a random copolymer, an alternating copolymer and a graft copolymer, and may also be another form.

The end group of the polymer compound is preferably a stable group since if a polymerization active group remains intact there, there is a possibility of a decrease in a light emitting property or luminance life when the polymer compound is used for fabrication of a light emitting device. The end group of the polymer compound is preferably a group conjugatively bonded to the main chain and includes, for example, groups bonding to an aryl group or a monovalent hetero ring group linking to the main chain of the polymer compound via a carbon-carbon bond.

"The alkyl group" may be any of linear or branched. The number of carbon atoms of the linear alkyl group, not including the number of carbon atoms of the substituent, is usually 1 to 50, preferably 1 to 20, and more preferably 1 to 10. The number of carbon atoms of the branched alkyl group, not including the number of carbon atoms of the substituent, is usually 3 to 50, preferably 3 to 20, and more preferably 4 to 10.

The alkyl group optionally has a substituent. The alkyl group includes, for example, a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group. Further, the alkyl group may also be a group obtained by substituting a part or all of hydrogen atoms in these groups with a cycloalkyl group, an alkoxy group, a cycloalkoxy group, an aryl group, a fluorine atom or the like. Such an alkyl group includes, for example, a trifluoromethyl group, a pentafluoroethyl group, a perfluorobutyl group, a perfluorohexyl group, a perfluorooctyl group, a 3-phenylpropyl group, a 3-(4-methylphenyl)propyl group, 3-(3,5-di-hexylphenyl)propyl group and a 6-ethyloxyhexyl group.

The number of carbon atoms of "the cycloalkyl group", not including the number of carbon atoms of the substituent, is usually 3 to 50, and preferably 4 to 10. The cycloalkyl group optionally has a substituent. The cycloalkyl group includes, for example, a cyclohexyl group and a methylcyclohexyl group.

The number of carbon atoms of "the alkylene group", not including the number of carbon atoms of the substituent, is usually 1 to 20, preferably 1 to 15, and more preferably 1 to 10. The alkylene group optionally has a substituent. The alkylene group includes, for example, a methylene group, an ethylene group, a propylene group, a butylene group, a hexylene group and an octylene group.

The number of carbon atoms of "the cycloalkylene group", not including the number of carbon atoms of the substituent, is usually 3 to 20, and preferably 4 to 10. The cycloalkylene group optionally has a substituent. The cycloalkylene group includes, for example, a cyclohexylene group.

"The aromatic hydrocarbon group" means a group obtained by removing from an aromatic hydrocarbon one or more hydrogen atoms bonding directly to atoms constituting the ring. The group obtained by removing from an aromatic hydrocarbon one hydrogen atom bonding directly to an atom constituting the ring is referred to also as "an aryl group". The group obtained by removing from an aromatic hydrocarbon two hydrogen atoms bonding directly to atoms constituting the ring is referred to also as "an arylene group".

The number of carbon atoms of the aromatic hydrocarbon group, not including the number of carbon atoms of the substituent, is usually 6 to 60, preferably 6 to 40, and more preferably 6 to 20.

"The aromatic hydrocarbon group" includes, for example, groups obtained by removing from a monocyclic aromatic hydrocarbon (including, for example, benzene) or a polycyclic aromatic hydrocarbon (including, for example, dicyclic aromatic hydrocarbons such as naphthalene, indene and the like; tricyclic aromatic hydrocarbons such as anthracene, phenanthrene, dihydrophenanthrene, fluorene and the like; tetracyclic aromatic hydrocarbons such as benzoanthracene, benzophenanthrene, benzofluorene, pyrene, fluoranthene and the like; pentacyclic aromatic hydrocarbons such as dibenzoanthracene, dibenzophenanthrene, dibenzofluorene, perylene, benzofluoranthene and the like; hexacyclic aromatic hydrocarbons such as spirobifluorene and the like; and, heptacyclic aromatic hydrocarbons such as benzospirobifluorene, acenaphthofluoranthene and the like) one or more hydrogen atoms bonding directly to atoms constituting the ring. The aromatic hydrocarbon group includes groups obtained by bonding a plurality of these groups. The aromatic hydrocarbon group optionally has a substituent.

"The alkoxy group" may be any of linear or branched. The number of carbon atoms of the linear alkoxy group, not including the number of carbon atoms of the substituent, is usually 1 to 40, and preferably 1 to 10. The number of carbon atoms of the branched alkoxy group, not including the number of carbon atoms of the substituent, is usually 3 to 40, and preferably 4 to 10.

The alkoxy group optionally has a substituent. The alkoxy group includes, for example, a methoxy group, an ethoxy group, an isopropyloxy group, a butyloxy group, a hexyloxy group, a 2-ethylhexyloxy group, a 3,7-dimethyloctyloxy group and a lauryloxy group.

The number of carbon atoms of "the cycloalkoxy group", not including the number of carbon atoms of the substituent, is usually 3 to 40, and preferably 4 to 10. The cycloalkoxy group optionally has a substituent. The cycloalkoxy group includes, for example, a cyclohexyloxy group.

The number of carbon atoms of "the aryloxy group", not including the number of carbon atoms of the substituent, is usually 6 to 60, preferably 6 to 40, and more preferably 6 to 20. The aryloxy group optionally has a substituent. The aryloxy group includes, for example, a phenoxy group, a naphthyloxy group, an anthracenyloxy group and a pyrenyloxy group.

"The hetero ring group" means a group obtained by removing from a heterocyclic compound one or more hydrogen atoms bonding directly to atoms constituting the ring. Of the hetero ring groups, "an aromatic hetero ring group" which is a group obtained by removing from an aromatic heterocyclic compound one or more hydrogen atoms bonding directly to atoms constituting the ring is preferable. The group obtained by removing from a heterocyclic compound p hydrogen atoms bonding directly to atoms constituting the ring (p represents an integer of 1 or more) is referred to also as "p-valent hetero ring group". The group obtained by removing from an aromatic heterocyclic compound p hydrogen atoms bonding directly to atoms constituting the ring is referred to also as "p-valent aromatic hetero ring group".

"The aromatic heterocyclic compound" includes, for example, compounds in which the hetero ring itself shows aromaticity such as azole, thiophene, furan, pyridine, diazabenzene, triazine, azanaphthalene, diazanaphthalene, carbazole and the like, and, compounds in which an aromatic ring is condensed to a hetero ring even if the hetero ring itself shows no aromaticity such as phenoxazine, phenothiazine, benzopyran and the like.

The number of carbon atoms of the hetero ring group, not including the number of carbon atoms of the substituent, is usually 1 to 60, preferably 2 to 40, and more preferably 3 to 20. The number of hetero atoms of the aromatic hetero ring group, not including the number of hetero atoms of the substituent, is usually 1 to 30, preferably 1 to 10, more preferably 1 to 5, and further preferably 1 to 3.

The hetero ring group includes, for example, groups obtained by removing from a monocyclic heterocyclic compound (including, for example, furan, thiophene, oxadiazole, pyrrole, diazole, triazole, tetrazole, pyridine, diazabenzene and triazine) or a polycyclic heterocyclic compound (including, for example, dicyclic heterocyclic compounds such as azanaphthalene, diazanaphthalene, benzofuran, benzothiophene, indole, benzodiazole, benzothiadiazole and the like; tricyclic heterocyclic compounds such as dibenzofuran, dibenzothiophene, dibenzoborole, dibenzosilole, dibenzophosphole, dibenzoselenophene, carbazole, azacarbazole, diazacarbazole, phenoxazine, phenothiazine, 9,10-dihydroacridine, 5,10-dihydrophenazine, phenazaborine, phenophosphazine, phenoselenazine, phenazasiline, azaanthracene, diazaanthracene, azaphenanthrene, diazaphenanthrene and the like; tetracyclic heterocyclic compounds such as hexaazatriphenylene, benzocarbazole, benzonaphthofurane, benzonaphthothiophene and the like; pentacyclic heterocyclic compounds such as dibenzocarbazole, indolocarbazole, indenocarbazole and the like; hexacyclic heterocyclic compounds such as carbazolocarbazole, benzoindolocarbazole, benzoindenocarbazole and the like; and, heptacyclic heterocyclic compounds such as dibenzoindolocarbazole and the like) one or more hydrogen atoms bonding directly to atoms constituting the ring. The hetero ring group includes groups obtained by bonding a plurality of these groups. The hetero ring group optionally has a substituent.

"The halogen atom" denotes a fluorine atom, a chlorine atom, a bromine atom or an iodine atom.

"The amino group" optionally has a substituent, and substituted amino groups (namely, secondary amino groups or tertiary amino groups, more preferably, tertiary amino groups) are preferred. The substituent which the amino group has is preferably an alkyl group, a cycloalkyl group, an aryl group or a monovalent hetero ring group. When a plurality of the substituents which the amino group has are present, they may be the same or different and may be combined together to form a ring together with nitrogen atoms to which they are attached.

The substituted amino group includes, for example, a dialkylamino group, a dicycloalkylamino group and a diarylamino group.

The amino group includes, for example, a dimethylamino group, a diethylamino group, a diphenylamino group, a bis(methylphenyl)amino group and a bis(3,5-di-tert-butylphenyl)amino group.

"The alkenyl group" may be any of linear or branched. The number of carbon atoms of the linear alkenyl group, not including the number of carbon atoms of the substituent, is usually 2 to 30, and preferably 3 to 20. The number of carbon atoms of the branched alkenyl group, not including the number of carbon atoms of the substituent, is usually 3 to 30, ,and preferably 4 to 20.

The number of carbon atoms of "the cycloalkenyl group", not including the number of carbon atoms of the substituent, is usually 3 to 30, and preferably 4 to 20.

The alkenyl group and the cycloalkenyl group optionally have a substituent. The alkenyl group includes, for example, a vinyl group, a 1-propenyl group, a 2-butenyl group, a 3-butenyl group, a 3-pentenyl group, a 4-pentenyl group, a 1-hexenyl group, a 5-hexenyl group and a 7-octenyl group, and groups obtained by substituting a part or all of hydrogen atoms in these groups with a substituent. The cycloalkenyl group includes, for example, a cyclohexenyl group, a cyclohexadienyl group, a cyclooctatrienyl group and a norbornylenyl group, and groups obtained by substituting a part or all of hydrogen atoms in these groups with a substituent.

"The alkynyl group" may be any of linear or branched. The number of carbon atoms of the alkynyl group, not including carbon atoms of the substituent, is usually 2 to 20, and preferably 3 to 20. The number of carbon atoms of the branched alkynyl group, not including carbon atoms of the substituent, is usually 4 to 30, and preferably 4 to 20.

The number of carbon atoms of "the cycloalkynyl group", not including carbon atoms of the substituent, is usually 4 to 30, and preferably 4 to 20.

The alkynyl group and the cycloalkynyl group optionally have a substituent. The alkynyl group includes, for example, an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and groups obtained by substituting a part or all of hydrogen atoms in these groups with a substituent. The cycloalkynyl group includes, for example, a cyclooctynyl group.

"The cross-linkable group" refers to a group capable of generating a new bond by being subjected to a heating treatment, an ultraviolet irradiation treatment, a near-ultraviolet irradiation treatment, a visible light irradiation treatment, an infrared irradiation treatment, a radical reaction and the like. As the cross-linkable group, cross-linkable groups selected from Group A of cross-linkable group (namely, groups represented by any of the (XL-1) to the (XL-19)) are preferred.

### (Group A of cross-linkable group)

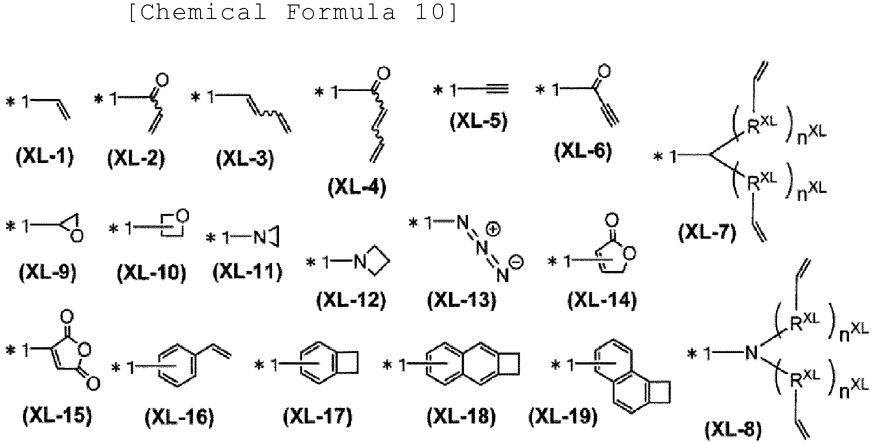

[wherein, R^{XL} represents a methylene group, an oxygen atom or a sulfur atom, and n^{XL} represents an integer of 0 to 5. When a plurality of R^{XL} are present, they may be the same or different. A plurality of n^{XL} may be the same or different. *1 represents a binding position. These cross-linkable groups optionally have a substituent. When a plurality of the substituents are present, they may be the same or different and may be combined together to form a ring together with carbon atoms to which they are attached.]

"The substituent" includes, for example, a halogen atom, a cyano group, an alkyl group, a cycloalkyl group, an aryl group, a monovalent hetero ring group, an alkoxy group, a cycloalkoxy group, an aryloxy group, an amino group, a substituted amino group, an alkenyl group, a cycloalkenyl group, an alkynyl group and a cycloalkynyl group. The substituent may be a cross-linkable group. When a plurality of the substituents are present, they may be combined together to form a ring together with atoms to which they are attached, but it is preferable that they do not form a ring.

In the present specification, calculation of the value of the absolute value of a difference between the energy level of the lowest triplet excited state and the energy level of the lowest singlet excited state (hereinafter, referred to also as "ΔE_{ST}"), is carried out by the following method. First, the ground state of a compound is structurally optimized by density-functional approach of B3LYP level. In this procedure, 6-31G* is used as the basis function. Using the resultant structurally optimized structure, ΔE_{ST} of the compound is calculated by B3LYP level time-dependent density-functional approach. In the case of containing an atom to which 6-31G* cannot be applied, LANL2DZ is used for the atom. Calculation is performed using Gaussian09, in the quantum chemistry calculation program.

### <Composition for light emitting device>

The composition for light emitting device of the present embodiment contains a metal complex represented by the formula (2), and a compound (B) having a condensed hetero ring skeleton (b).

The metal complex represented by the formula (2) and the compound (B) each may be contained singly or in combination of two or more in the composition for light emitting device of the present embodiment.

In the composition for light emitting device of the present embodiment, the metal complex represented by the formula (2) and the compound (B) preferably interact physically, chemically or electrically. By this interaction, it is possible to improve or adjust, for example, the light emitting property, the charge transporting property or the charge injection property of the composition for light emitting device of the present embodiment.

In the composition for light emitting device of the present embodiment, if a light emitting material is described as an example, the metal complex represented by the formula (2) and the compound (B) interact electrically to transfer electrical energy efficiently from the compound (B) to the metal complex represented by the formula (2), accordingly, the metal complex represented by the formula (2) can be allowed to emit light more efficiently and the light emitting device of the present embodiment is more excellent in light emission efficiency.

From the above-described viewpoint, it is preferable that the lowest excited triplet state (T₁) of the compound (B) has the energy level higher than that of the lowest excited triplet state (T₁) of the metal complex represented by the formula (2), since the light emitting device of the present embodiment is more excellent in light emission efficiency.

In the composition for light emitting device of the present embodiment, the content of the metal complex represented by the formula (2) is usually 0.1 to 99.9 parts by mass, when the sum of the compound (B) and the metal complex represented by the formula (2) is taken as 100 parts by mass, and it is preferably 1 to 99 parts by mass, more preferably 10 to 97 parts by mass, further preferably 30 to 95 parts by mass, particularly preferably 50 to 90 parts by mass, and especially preferably 70 to 90 parts by mass, since the light emitting device of the present embodiment is more excellent in light emission efficiency.

### [Compound (B)]

The compound (B) is a compound having a condensed hetero ring skeleton (b) containing a boron atom and at least one selected from the group consisting of an oxygen atom, a sulfur atom, a selenium atom, an sp³ carbon atom and a nitrogen atom in the ring.

In the compound (B), if the condensed hetero ring skeleton (b) contain nitrogen atoms, it is preferable that at least one of nitrogen atoms contained in the condensed hetero ring skeleton (b) is a nitrogen atom not forming a double bond, and it is more preferable that all nitrogen atoms contained in the condensed hetero ring skeleton (b) are nitrogen atoms not forming a double bond.

The number of carbon atoms of the condensed hetero ring skeleton (b), not including the number of carbon atoms of the substituent, is usually 1 to 60, preferably 5 to 40, and more preferably 10 to 25.

The number of hetero atoms of the condensed hetero ring skeleton (b), not including the number of hetero atoms of the substituent, is usually 2 to 30, preferably 2 to 15, more preferably 2 to 10, further preferably 2 to 5, and particularly preferably 2 or 3.

The number of boron atoms of the condensed hetero ring skeleton (b), not including the number of boron atoms of the substituent, is usually 1 to 10, preferably 1 to 5, more preferably 1 to 3, and further preferably 1.

The total number of an oxygen atom, a sulfur atom, a selenium atom, an sp³ carbon atom and a nitrogen atom of the condensed hetero ring skeleton (b), not including the number of atoms of the substituent, is usually 1 to 20, preferably 1 to 10, more preferably 1 to 5, further preferably 1 to 3, and particularly preferably 2.

The condensed hetero ring skeleton (b) preferably contains a boron atom and at least one selected from the group consisting of an oxygen atom, a sulfur atom and a nitrogen atom in the ring, more preferably contains a boron atom and a nitrogen atom in the ring, and further preferably contains a boron atom and a nitrogen atom not forming a double bond in the ring, since the light emitting device of the present embodiment is more excellent in light emission efficiency.

The condensed hetero ring skeleton (b) is preferably a tri to dodecacyclic condensed hetero ring skeleton, more preferably a tri to hexacyclic condensed hetero ring skeleton, and further preferably a pentacyclic condensed hetero ring skeleton, since the light emitting device of the present embodiment is more excellent in light emission efficiency.

The condensed hetero ring skeleton (b) can also be referred to as a compound having a hetero ring group (b') containing a condensed hetero ring skeleton (b).

The hetero ring group (b') may be a group obtained by removing from a polycyclic heterocyclic compound containing a boron atom and at least one selected from the group consisting of an oxygen atom, a sulfur atom, a selenium atom, an sp³ carbon atom and a nitrogen atom in the ring one or more hydrogen atoms bonding directly to atoms constituting the ring, and the group optionally has a substituent. In the hetero ring group (b'), the polycyclic heterocyclic compound is preferably a polycyclic heterocyclic compound containing a boron atom and at least one selected from the group consisting of an oxygen atom, a sulfur atom and a nitrogen atom in the ring, more preferably a polycyclic heterocyclic compound containing a boron atom and a nitrogen atom in the ring, and further preferably a polycyclic heterocyclic compound containing a boron atom and a nitrogen atom not forming a double bond in the ring. In the hetero ring group (b'), the polycyclic heterocyclic compound is preferably a tri to dodecacyclic heterocyclic compound, more preferably a tri to hexacyclic heterocyclic compound, and further preferably a pentacyclic heterocyclic compound.

The substituent which a hetero ring group (b') optionally has is preferably a halogen atom, a cyano group, an alkyl group, a cycloalkyl group, an alkoxy group, a cycloalkoxy group, an aryl group, a monovalent hetero ring group or a substituted amino group, more preferably an alkyl group, a cycloalkyl group, an alkoxy group, a cycloalkoxy group, an aryl group, a monovalent hetero ring group or a substituted amino group, and further preferably an alkyl group, an aryl group or a substituted amino group, and these groups optionally further have a substituent.

The aryl group as the substituent which a hetero ring group (b') optionally has is preferably a group obtained by removing from a monocyclic or dicyclic to hexacyclic aromatic hydrocarbon one hydrogen atom bonding directly to an atom constituting the ring, more preferably a group obtained by removing from a monocyclic, dicyclic or tricyclic aromatic hydrocarbon one hydrogen atom bonding directly to an atom constituting the ring, further preferably a group obtained by removing from benzene, naphthalene, anthracene, phenanthrene or fluorene one hydrogen atom bonding directly to an atom constituting the ring, and particularly preferably a phenyl group, and these groups optionally have a substituent.

The monovalent hetero ring group as the substituent which a hetero ring group (b') optionally has is preferably a group obtained by removing from a monocyclic or dicyclic to hexacyclic heterocyclic compound one hydrogen atom bonding directly to an atom constituting the ring, more preferably a group obtained by removing from a monocyclic, dicyclic or tricyclic heterocyclic compound one hydrogen atom bonding directly to an atom constituting the ring, further preferably a group obtained by removing from pyridine, diazabenzene, triazine, azanaphthalene, diazanaphthalene, carbazole, dibenzofuran, dibenzothiophene, phenoxazine or phenothiazine one hydrogen atom bonding directly to an atom constituting the ring, and particularly preferably a group obtained by removing from pyridine, diazabenzene or triazine one hydrogen atom bonding directly to an atom constituting the ring, and these groups optionally have a substituent.

In the substituted amino group as the substituent which a hetero ring group (b') optionally has, the substituent which the amino group has is preferably an aryl group or a monovalent hetero ring group, and more preferably an aryl group, and these groups optionally further have a substituent. The examples and preferable ranges of the aryl group and the monovalent hetero ring group as the substituent which the amino group has are the same as the examples and preferable ranges of the aryl group and the monovalent hetero ring group as the substituent which a hetero ring group (b') optionally has.

The substituent which the substituent which a hetero ring group (b') optionally has optionally further has is preferably a halogen atom, an alkyl group, a cycloalkyl group, an alkoxy group, a cycloalkoxy group, an aryl group, a monovalent hetero ring group or a substituted amino group, more preferably an alkyl group, a cycloalkyl group, an aryl group, a monovalent hetero ring group or a substituted amino group, and further preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which a hetero ring group (b') optionally has optionally further has are the same as the examples and preferable ranges of the aryl group, the monovalent hetero ring group and the substituted amino group as the substituent which a hetero ring group (b') optionally has, respectively.

"The nitrogen atom not forming a double bond" means a nitrogen atom that is single-bonded to each of the other three atoms.

The phrase "containing a nitrogen atom not forming a double bond in the ring" means that a group represented by -N(-R^{N})- (wherein, R^{N} represents a hydrogen atom or a substituent) or the formula: 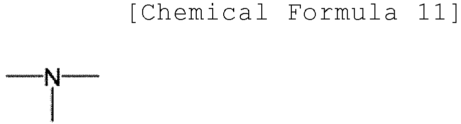 is contained in the ring.

The compound (B) is preferably a thermally activated delayed fluorescent (TADF) compound, since the light emitting device of the present embodiment is more excellent in light emission efficiency.

ΔE_{ST} of the compound (B) may be 2.0 eV or less, may be 1.5 eV or less, may be 1.0 eV or less, may be 0.80 eV or less, or may be 0.60 eV or less, and it is preferably 0.50 eV or less, since the light emitting device of the present embodiment is more excellent in light emission efficiency. Meanwhile, ΔEST of the compound (B) may be 0.001 eV or more, may be 0.01 eV or more, may be 0.10 eV or more, may be 0.20 eV or more, may be 0.30 eV or more, or may be 0.40 eV or more.

The compound (B) is preferably a low molecular weight compound.

The molecular weight of the compound (B) is preferably 1×10² to 5×10³, more preferably 2×10² to 3×10³, further preferably 3×10² to 1.5×10³, and particularly preferably 4×10² to 1×10³.

The compound (B) is preferably a compound represented by the formula (1-1), the formula (1-2) or the formula (1-3), more preferably a compound represented by the formula (1-2) or the formula (1-3), and further preferably a compound represented by the formula (1-2), since the light emitting device of the present embodiment is more excellent in light emission efficiency.

Ar¹, Ar² and Ar³ are each a group obtained by removing from a monocyclic, dicyclic or tricyclic aromatic hydrocarbon or a monocyclic, dicyclic or tricyclic heterocyclic compound one or more hydrogen atoms bonding directly to atoms constituting the ring, more preferably a group obtained by removing from a monocyclic aromatic hydrocarbon or a monocyclic heterocyclic compound one or more hydrogen atoms bonding directly to atoms constituting the ring, further preferably a group obtained by removing from benzene, pyridine or diazabenzene one or more hydrogen atoms bonding directly to atoms constituting the ring, and particularly preferably a group obtained by removing from benzene one or more hydrogen atoms bonding directly to atoms constituting the ring, since the light emitting device of the present embodiment is more excellent in light emission efficiency, and these groups optionally have a substituent.

The examples and preferable ranges of the substituent which Ar¹, Ar² and Ar³ optionally have are the same as the examples and preferable ranges of the substituent which a hetero ring group (b') optionally has.

Y¹ is preferably an oxygen atom, a sulfur atom, a group represented by -N(Ry)- or a methylene group, more preferably an oxygen atom, a sulfur atom or a group represented by -N(Ry)-, and further preferably a group represented by -N(Ry)-, and these groups optionally have a substituent.

Y² and Y³ are each preferably a single bond, an oxygen atom, a sulfur atom, a group represented by - N(Ry)- or a methylene group, more preferably a single bond, an oxygen atom, a sulfur atom or a group represented by -N(Ry)-, further preferably an oxygen atom, a sulfur atom or a group represented by -N(Ry)-, and particularly preferably a group represented by - N(Ry)-, and these groups optionally have a substituent.

It is preferable that all of Y¹, Y² and Y³ represent an oxygen atom, a sulfur atom or a group represented by -N(Ry)-, and it is more preferable that all of Y¹, Y² and Y³ represent a group represented by - N(Ry)-, since the light emitting device of the present embodiment is more excellent in light emission efficiency.

The examples and preferable ranges of the substituent which Y¹, Y² and Y³ optionally have are the same as the examples and preferable ranges of the substituent which a hetero ring group (b') optionally has.

Ry is preferably an alkyl group, a cycloalkyl group, an aryl group or a monovalent hetero ring group, more preferably an aryl group or a monovalent hetero ring group, further preferably an aryl group, and these groups optionally have a substituent.

The examples and preferable ranges of the aryl group and the monovalent hetero ring group represented by Ry are the same as the examples and preferable ranges of the aryl group and the monovalent hetero ring group as the substituent which a hetero ring group (b') optionally has, respectively.

The examples and preferable ranges of the substituent which Ry optionally has are the same as the examples and preferable ranges of the substituent which a hetero ring group (b') optionally has.

Ry may be bonded directly or via a connecting group to Ar¹, Ar² or Ar³, but it is preferable that it is not bonded. The connecting group includes, for example, a group represented by -O-, a group represented by -S-, a group represented by -N(Ry)-, an alkylene group, a cycloalkylene group, an arylene group and a divalent hetero ring group, and is preferably a group represented by -O-, a group represented by -S-, a group represented by -N(Ry)- or a methylene group, and these groups optionally have a substituent.

As the compound (B), compounds represented by the following formulae and compounds B1 to B3 described later are exemplified.

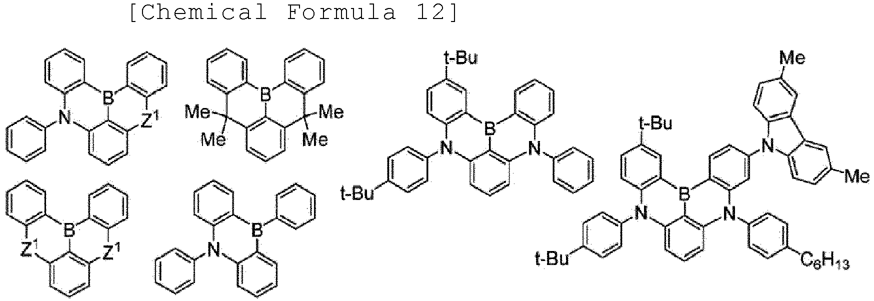

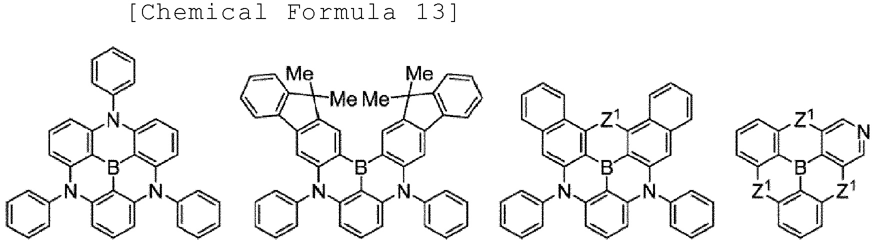

In the formulae, Z¹ represents an oxygen atom or a sulfur atom.

### [Metal complex represented by the formula (2)]

The metal complex represented by the formula (2) is usually a metal complex exhibiting a phosphorescent property at room temperature, and preferably a metal complex exhibiting light emission from the triplet excited state at room temperature.

The maximum peak wavelength of the light emission spectrum of the metal complex represented by the formula (2) is preferably 495 nm or more and 750 nm or less, more preferably 500 nm or more and 680 nm or less, further preferably 505 nm or more and 660 nm or less, and particularly preferably 510 nm or more and 640 nm or less.

The maximum peak wavelength of the light emission spectrum of the metal complex can be evaluated by dissolving the metal complex in an organic solvent such as xylene, toluene, chloroform, tetrahydrofuran and the like to prepare a dilute solution (1×10⁻⁶ to 1×10⁻³% by mass), and measuring the PL spectrum of the dilute solution at room temperature. As the organic solvent for dissolving the metal complex, xylene is preferable.

M² is preferably an iridium atom or a platinum atom, and more preferably an iridium atom, since the light emitting device of the present embodiment is more excellent in light emission efficiency.

When M² is a rhodium atom or an iridium atom, n³ is preferably 2 or 3, and more preferably 3.

When M² is a palladium atom or a platinum atom, n³ is preferably 2.

E^{L} is preferably a carbon atom. When a plurality of E^{L} are present, it is preferable that they are the same.

The number of carbon atoms of the aromatic hetero ring containing a 6-membered ring represented by Ring L¹, not including the number of carbon atoms of the substituent, is usually 1 to 60, preferably 2 to 30, and further preferably 3 to 15. The aromatic hetero ring containing a 6-membered ring represented by Ring L¹ includes, for example, aromatic hetero rings having a nitrogen atom in the ring and containing a 6-membered ring, among aromatic hetero rings which an aromatic heterocyclic compound has exemplified in the section of the hetero ring group described above.

Ring L¹ is preferably an aromatic hetero ring containing a 6-membered ring having 1 or more and 4 or less nitrogen atoms as the constituent atom, more preferably a pyridine ring, a diazabenzene ring, an azanaphthalene ring or a diazanaphthalene ring, further preferably a pyridine ring, a quinoline ring or an isoquinoline ring, particularly preferably a pyridine ring or a quinoline ring, and especially preferably a pyridine ring, since the light emitting device of the present embodiment is more excellent in light emission efficiency, and these rings optionally have a substituent.

When a plurality of Ring L¹ are present, it is preferable that at least two of the plurality of Ring L¹ are identical, and it is more preferable that all of the plurality of Ring L¹ are identical, since a metal complex represented by the formula (2) can be synthesized easily.

The number of carbon atoms of the aromatic hydrocarbon ring represented by Ring L², not including the number of carbon atoms of the substituent, is usually 6 to 60, preferably 6 to 30, and further preferably 6 to 18.

The aromatic hydrocarbon ring represented by Ring L² includes, for example, aromatic hydrocarbon rings which an aromatic hydrocarbon has exemplified in the section of the aromatic hydrocarbon group described above.

The aromatic hydrocarbon ring represented by Ring L² is preferably a monocyclic, dicyclic or tricyclic aromatic hydrocarbon ring, more preferably a benzene ring, a naphthalene ring, a fluorene ring, a phenanthrene ring or a dihydrophenanthrene ring, further preferably a benzene ring, a fluorene ring or a dihydrophenanthrene ring, and particularly preferably a benzene ring, and these rings optionally have a substituent.

The number of carbon atoms of the aromatic hetero ring represented by Ring L², not including the number of carbon atoms of the substituent, is usually 1 to 60, preferably 2 to 30, and more preferably 3 to 15. The number of hetero atoms of the aromatic hetero ring represented by Ring L², not including the number of hetero atoms of the substituent, is usually 1 to 30, preferably 1 to 10, and more preferably 1 to 3.

The aromatic hetero ring represented by Ring L² includes, for example, aromatic hetero rings which an aromatic heterocyclic compound has exemplified in the section of the hetero ring group described above.

The aromatic hetero ring represented by Ring L² is preferably a monocyclic, dicyclic or tricyclic aromatic hetero ring, more preferably a pyridine ring, a diazabenzene ring, an azanaphthalene ring, a diazanaphthalene ring, an indole ring, a benzofuran ring, a benzothiophene ring, a carbazole ring, an azacarbazole ring, a diazacarbazole ring, a dibenzofuran ring or a dibenzothiophene ring, further preferably a pyridine ring, a diazabenzene ring, a carbazole ring, a dibenzofuran ring or a dibenzothiophene ring, and particularly preferably a pyridine ring or a diazabenzene ring, and these rings optionally have a substituent.

Ring L² is preferably a benzene ring, a pyridine ring or a diazabenzene ring, more preferably a benzene ring, since the light emitting device of the present embodiment is more excellent in light emission efficiency, and these rings optionally have a substituent.

When a plurality of Ring L² are present, it is preferable that at least two of the plurality of Ring L² are identical, and it is more preferable that all of the plurality of Ring L² are identical, since a metal complex represented by the formula (2) can be synthesized easily.

It is preferable that Ring L¹ is a pyridine ring, a diazabenzene ring, an azanaphthalene ring or a diazanaphthalene ring, and Ring L² is a benzene ring, a pyridine ring or a diazabenzene ring, it is more preferable that Ring L¹ is a pyridine ring, a quinoline ring or an isoquinoline ring, and Ring L² is a benzene ring, it is further preferable that Ring L¹ is a pyridine ring or a quinoline ring, and Ring L² is a benzene ring, and it is particularly preferable that Ring L¹ is a pyridine ring, and Ring L² is a benzene ring, since the light emitting device of the present embodiment is more excellent in light emission efficiency. These rings optionally have a substituent.

The phrase "At least one of Ring L¹ and Ring L² has a group represented by the formula (1-T) as a substituent" means that a group represented by the formula (1-T) is bonded directly to at least one of atoms (preferably, carbon atom or nitrogen atom) constituting Ring L¹ and Ring L².

In the metal complex represented by the formula (2), when a plurality of Ring L¹ and Ring L² are present, at least one of the plurality of Ring L¹ and Ring L² may have a group represented by the formula (1-T), and it is preferable that all of the plurality of Ring L¹, all of the plurality of Ring L², or all of the plurality of Ring L¹ and Ring L² have a group represented by the formula (1-T), and it is more preferable that all of the plurality of Ring L¹, or all of the plurality of Ring L² have a group represented by the formula (1-T), since the light emitting device of the present embodiment is more excellent in light emission efficiency.

In the metal complex represented by the formula (2), the number of groups represented by the formula (1-T) which at least one of Ring L¹ and Ring L² has is usually 1 to 5, and it is preferably 1 to 3, more preferably 1 or 2, and further preferably 1, since the metal complex represented by the formula (2) can be synthesized easily.

In the metal complex represented by the formula (2), when M² is a rhodium atom or an iridium atom, the total number of groups represented by the formula (1-T) which Ring L¹ and Ring L² have is usually 1 to 30, and it is preferably 1 to 18, more preferably 2 to 12, and further preferably 3 to 6, since the light emitting device of the present embodiment is more excellent in light emission efficiency.

In the metal complex represented by the formula (2), when M² is a palladium atom or a platinum atom, the total number of groups represented by the formula (1-T) which Ring L¹ and Ring L² have is usually 1 to 20, and it is preferably 1 to 12, more preferably 1 to 8, and further preferably 2 to 4, since the light emitting device of the present embodiment is more excellent in light emission efficiency.

The substituent which Ring L¹ and Ring L² optionally have is preferably a group represented by the formula (1-T), since the light emitting device of the present embodiment is more excellent in light emission efficiency.

In the substituent which Ring L¹ and Ring L² optionally have, the substituent other than the group represented by the formula (1-T) is preferably a cyano group, an alkenyl group or a cycloalkenyl group, and these groups optionally further have a substituent. The examples and preferable ranges of the substituent which the substituent other than the group represented by the formula (1-T) optionally further has are the same as the examples and preferable ranges of the substituent which R^{1T} optionally has described later.

### [Group represented by the formula (1-T)]

The aryl group represented by R^{1T} is preferably a group obtained by removing from a monocyclic, dicyclic or tricyclic aromatic hydrocarbon one hydrogen atom bonding directly to an atom constituting the ring, more preferably a phenyl group, naphthyl group or fluorenyl group, and further preferably a phenyl group, and these groups optionally have a substituent.

The monovalent hetero ring group represented by R^{1T} is preferably a group obtained by removing from a monocyclic, dicyclic or tricyclic heterocyclic compound one hydrogen atom bonding directly to an atom constituting the ring, more preferably a group obtained by removing from pyridine, diazabenzene, triazine, azanaphthalene, diazanaphthalene, carbazole, dibenzofuran or dibenzothiophene one hydrogen atom bonding directly to an atom constituting the ring, further preferably a group obtained by removing from pyridine, diazabenzene or triazine one hydrogen atom bonding directly to an atom constituting the ring, and these groups optionally have a substituent.

In the substituted amino group represented by R^{1T}, the substituent which the amino group has is preferably an aryl group or a monovalent hetero ring group, and more preferably an aryl group, and these groups optionally further have a substituent. The examples and preferable ranges of the aryl group as the substituent which the amino group has are the same as the examples and preferable ranges of the aryl group represented by R^{1T}. The examples and preferable ranges of the monovalent hetero ring group as the substituent which the amino group has are the same as the examples and preferable ranges of the monovalent hetero ring group represented by R^{1T}.

In at least one group represented by the formula (1-T), R^{1T} is preferably an alkyl group, a cycloalkyl group, an aryl group, a monovalent hetero ring group or a substituted amino group, R^{1T} is more preferably an aryl group, a monovalent hetero ring group or a substituted amino group, R^{1T} is further preferably an aryl group or a monovalent hetero ring group, and R^{1T} is particularly preferably an aryl group, since the light emitting device of the present embodiment is more excellent in light emission efficiency, and these groups optionally have a substituent.

When Ring L¹ is an isoquinoline ring, in at least one group represented by the formula (1-T), R^{1T} is preferably an aryl group, a monovalent hetero ring group or a substituted amino group, R^{1T} is more preferably an aryl group or a monovalent hetero ring group, and R^{1T} is further preferably an aryl group, since the light emitting device of the present embodiment is further excellent in light emission efficiency, and these groups optionally have a substituent.

When Ring L¹ is an aromatic hetero ring containing a 6-membered ring other than an isoquinoline ring (preferably a pyridine ring, a diazabenzene ring, a quinoline ring or a diazanaphthalene ring, more preferably a pyridine ring or a quinoline ring, and further preferably a pyridine ring), in at least one group represented by the formula (1-T), R^{1T} is preferably an alkyl group, a cycloalkyl group, an aryl group, a monovalent hetero ring group or a substituted amino group, R^{1T} is more preferably an alkyl group, a cycloalkyl group, an aryl group or a monovalent hetero ring group, R^{1T} is further preferably an aryl group or a monovalent hetero ring group, and R^{1T} is particularly preferably an aryl group, since the light emitting device of the present embodiment is further excellent in light emission efficiency, and these groups optionally have a substituent.

R^{1T} is preferably an alkyl group, a cycloalkyl group, an aryl group, a monovalent hetero ring group or a substituted amino group, more preferably an aryl group, a monovalent hetero ring group or a substituted amino group, further preferably an aryl group or a monovalent hetero ring group, and particularly preferably an aryl group, since the light emitting device of the present embodiment is more excellent in light emission efficiency, and these groups optionally have a substituent.

When Ring L¹ is an isoquinoline ring, R^{1T} is preferably an aryl group, a monovalent hetero ring group or a substituted amino group, more preferably an aryl group or a monovalent hetero ring group, and further preferably an aryl group, since the light emitting device of the present embodiment is further excellent in light emission efficiency, and these groups optionally have a substituent.

When Ring L¹ is an aromatic hetero ring containing a 6-membered ring other than an isoquinoline ring (preferably a pyridine ring, a diazabenzene ring, a quinoline ring or a diazanaphthalene ring, more preferably a pyridine ring or a quinoline ring, and further preferably a pyridine ring), R^{1T} is preferably an alkyl group, a cycloalkyl group, an aryl group, a monovalent hetero ring group or a substituted amino group, more preferably an alkyl group, a cycloalkyl group, an aryl group or a monovalent hetero ring group, further preferably an aryl group or a monovalent hetero ring group, and particularly preferably an aryl group, since the light emitting device of the present embodiment is further excellent in light emission efficiency, and these groups optionally have a substituent.

The substituent which R^{1T} optionally has is preferably an alkyl group, a cycloalkyl group, an alkoxy group, a cycloalkoxy group, an aryl group, a monovalent hetero ring group, a substituted amino group or a fluorine atom, more preferably an alkyl group, a cycloalkyl group, an aryl group, a monovalent hetero ring group or a substituted amino group, and further preferably an alkyl group, a cycloalkyl group or an aryl group, and these groups optionally further have a substituent.

The examples and preferable ranges of the aryl group, the monovalent hetero ring group and the substituted amino group as the substituent which R^{1T} optionally has are the same as the examples and preferable ranges of the aryl group, the monovalent hetero ring group and the substituted amino group represented by R^{1T}, respectively.

The substituent which the substituent which R^{1T} optionally has optionally further has is preferably an alkyl group, a cycloalkyl group, an alkoxy group, a cycloalkoxy group, an aryl group, a monovalent hetero ring group, a substituted amino group or a fluorine atom, more preferably an alkyl group, a cycloalkyl group, an aryl group, a monovalent hetero ring group or a substituted amino group, and further preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which R^{1T} optionally has optionally further has are the same as the examples and preferable ranges of the aryl group, the monovalent hetero ring group and the substituted amino group represented by R^{1T}, respectively.

### [Anionic bidentate ligand]

The anionic bidentate ligand represented by A³-G²-A⁴ includes, for example, ligands represented by the following formulae. The anionic bidentate ligand represented by A³-G²-A⁴ is different from a ligand of which number is defined by the suffix n³.

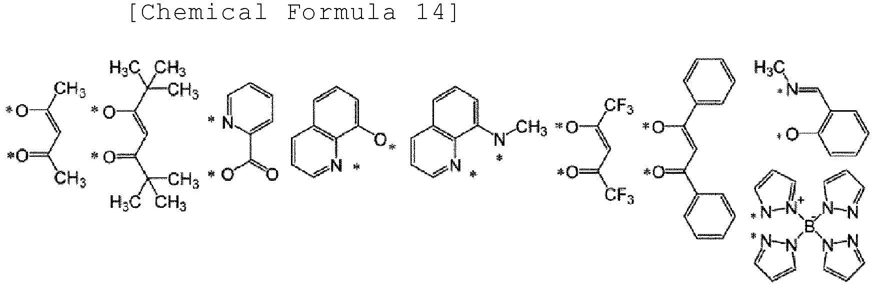

In the formulae, * represents a site binding to M².

The metal complex represented by the formula (2) includes, for example, metal complexes represented by the following formulae, metal complexes G2 to G4 described later and metal complexes R3 to R7 described later.

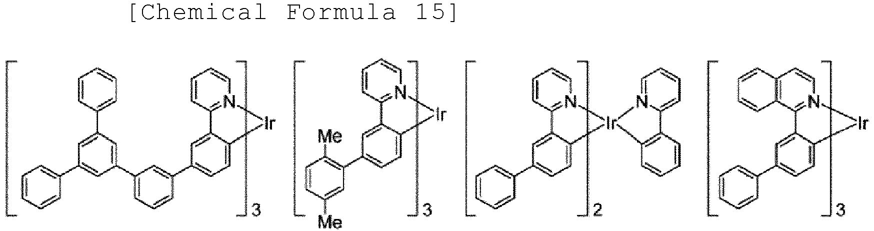

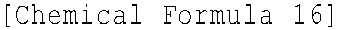

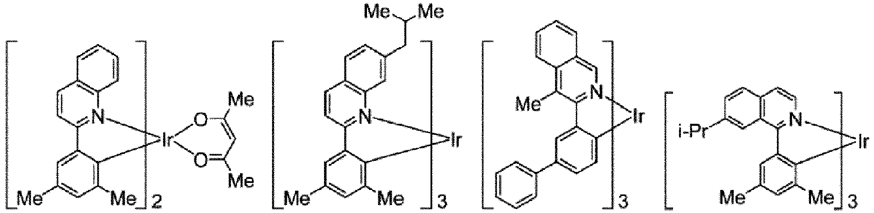

### [Host material]

It is preferable that the composition for light emitting device of the present embodiment further contains a host material having at least one function selected from hole injectability, hole transportability, electron injectability and electron transportability, since the light emitting device of the present embodiment is more excellent in light emission efficiency. The composition for light emitting device of the present embodiment may contain the host material singly or in combination of two or more. However, the host material is different from the compound (B). Further, the host material is different from the metal complex represented by the formula (2).

When the composition for light emitting device of the present embodiment further contains a host material, the content of the host material is usually 1 to 99.99 parts by mass, preferably 5 to 99.9 parts by mass, more preferably 10 to 99 parts by mass, further preferably 30 to 97 parts by mass, particularly preferably 50 to 95 parts by mass, and especially preferably 60 to 90 parts by mass, when the sum of the compound (B), the metal complex represented by the formula (2) and the host material is taken as 100 parts by mass.

When the composition for light emitting device of the present embodiment further contains a host material, it is preferable that the host material, the compound (B) and the metal complex represented by the formula (2) interact physically, chemically or electrically. By this interaction, it is possible to improve or adjust, for example, the light emitting property, the charge transporting property or the charge injection property of the composition for light emitting device of the present embodiment.

When the composition for light emitting device of the present embodiment further contains a host material, if a light emitting material is described as an example, the host material, the compound (B) and the metal complex represented by the formula (2) interact electrically to transfer electrical energy efficiently from the host material to the compound (B) and further transfer electrical energy efficiently from the compound (B) to the metal complex represented by the formula (2), accordingly, the metal complex represented by the formula (2) can be allowed to emit light more efficiently and the light emitting device of the present embodiment is more excellent in light emission efficiency.

From the above-described viewpoint, it is preferable that the lowest excited triplet state (T₁) of the host material has energy level higher than that of the lowest excited triplet state (T₁) of the metal complex represented by the formula (2) and the compound (B), since the light emitting device of the present embodiment is more excellent in light emission efficiency. Further, it is preferable that the lowest excited singlet state (S₁) of the host material has energy level higher than that of the lowest excited singlet state (S₁) of the compound (B), since the light emitting device of the present embodiment is more excellent in light emission efficiency.

The host material is preferably one showing solubility in a solvent which is capable of dissolving the metal complex represented by the formula (2) and the compound (B), since the light emitting device of the present embodiment can be fabricated by a wet method.

The host material is classified into low molecular weight compounds (low molecular weight host) and polymer compounds (polymer host), and the composition for light emitting device of the present embodiment may contain any host material. The host material which may be contained in the composition for light emitting device of the present embodiment is preferably a low molecular weight compound, since the light emitting device of the present embodiment is more excellent in light emission efficiency.

The polymer host includes, for example, polymer compounds as a hole transporting material described later and polymer compounds as an electron transporting material described later.

The low molecular weight host is preferably a compound represented by the formula (H-1), since the light emitting device of the present embodiment is more excellent in light emission efficiency. The compound represented by the formula (H-1) is preferably a compound having no condensed hetero ring skeleton (b) in the compound.

The molecular weight of the compound represented by the formula (H-1) is preferably 1×10² to 5×10³, more preferably 2×10² to 3×10³, further preferably 3×10² to 1.5×10³, and particularly preferably 4×10² to 1×10³.

The aryl group represented by Ar^{H1} and Ar^{H2} is preferably a group obtained by removing from a monocyclic or di to hexacyclic aromatic hydrocarbon one hydrogen atom bonding directly to an atom constituting the ring, more preferably a group obtained by removing from a monocyclic or di to tetracyclic aromatic hydrocarbon one hydrogen atom bonding directly to an atom constituting the ring, and further preferably a group obtained by removing from benzene, naphthalene, fluorene, phenanthrene or triphenylene one hydrogen atom bonding directly to an atom constituting the ring, and these groups optionally have a substituent.

The arylene group represented by L^{H1} is preferably a group obtained by removing from a monocyclic or di to hexacyclic aromatic hydrocarbon two hydrogen atoms bonding directly to atoms constituting the ring, more preferably a group obtained by removing from a monocyclic or di to tetracyclic aromatic hydrocarbon two hydrogen atoms bonding directly to atoms constituting the ring, and further preferably a group obtained by removing from benzene, naphthalene, fluorene, phenanthrene or triphenylene two hydrogen atoms bonding directly to atoms constituting the ring, and these groups optionally have a substituent.

The monovalent hetero ring group represented by Ar^{H1} and Ar^{H2} is preferably a group obtained by removing from a heterocyclic compound containing no condensed hetero ring skeleton (b) one hydrogen atom bonding directly to an atom constituting the ring, and this group optionally has a substituent. The heterocyclic compound containing no condensed hetero ring skeleton (b), in the monovalent hetero ring group represented by Ar^{H1} and Ar^{H2}, includes heterocyclic compounds not containing a boron atom and a nitrogen atom in the ring, among heterocyclic compounds described in the section of the hetero ring group described above. The monovalent hetero ring group represented by Ar^{H1} and Ar^{H2} is preferably a group obtained by removing from a monocyclic or di to hexacyclic heterocyclic compound (preferably, a monocyclic or di to hexacyclic heterocyclic compound containing no condensed hetero ring skeleton (b)) one hydrogen atom bonding directly to an atom constituting the ring, more preferably a group obtained by removing from a monocyclic, dicyclic, tricyclic or pentacyclic heterocyclic compound (preferably, a monocyclic, dicyclic, tricyclic or pentacyclic heterocyclic compound containing no condensed hetero ring skeleton (b)) one hydrogen atom bonding directly to an atom constituting the ring, further preferably a group obtained by removing from pyridine, diazabenzene, triazine, azanaphthalene, diazanaphthalene, carbazole, dibenzofuran, dibenzothiophene, phenoxazine, phenothiazine, dibenzocarbazole, indolocarbazole or indenocarbazole one hydrogen atom bonding directly to an atom constituting the ring, and particularly preferably a group obtained by removing from pyridine, diazabenzene, triazine, azanaphthalene, diazanaphthalene, carbazole, dibenzofuran or dibenzothiophene one hydrogen atom bonding directly to an atom constituting the ring, and these groups optionally have a substituent.

The divalent hetero ring group represented by L^{H1} is preferably a group obtained by removing from a heterocyclic compound containing no condensed hetero ring skeleton (b) two hydrogen atoms bonding directly to atoms constituting the ring. The heterocyclic compound containing no condensed hetero ring skeleton (b), in the divalent hetero ring group represented by L^{H1}, includes heterocyclic compounds not containing a boron atom and a nitrogen atom in the ring, among heterocyclic compounds described in the section of the hetero ring group described above. The divalent hetero ring group represented by L^{H1} is preferably a group obtained by removing from a monocyclic or di to hexacyclic heterocyclic compound (preferably, a monocyclic or di to hexacyclic heterocyclic compound containing no condensed hetero ring skeleton (b)) two hydrogen atoms bonding directly to atoms constituting the ring, more preferably a group obtained by removing from a monocyclic, dicyclic, tricyclic or pentacyclic heterocyclic compound (preferably, a monocyclic, dicyclic, tricyclic or pentacyclic heterocyclic compound containing no condensed hetero ring skeleton (b)) two hydrogen atoms bonding directly to atoms constituting the ring, further preferably a group obtained by removing from pyridine, diazabenzene, triazine, azanaphthalene, diazanaphthalene, carbazole, dibenzofuran, dibenzothiophene, phenoxazine, phenothiazine, dibenzocarbazole, indolocarbazole or indenocarbazole two hydrogen atoms bonding directly to atoms constituting the ring, and particularly preferably a group obtained by removing from pyridine, diazabenzene, triazine, azanaphthalene, diazanaphthalene, carbazole, dibenzofuran or dibenzothiophene two hydrogen atoms bonding directly to atoms constituting the ring, and these groups optionally have a substituent.

In the substituted amino group represented by Ar^{H1} and Ar^{H2}, the substituent which the amino group has is preferably an aryl group or a monovalent hetero ring group, and more preferably an aryl group, and these groups optionally further have a substituent. The examples and preferable ranges of the aryl group as the substituent which the amino group has are the same as the examples and preferable ranges of the aryl group represented by Ar^{H1} and Ar^{H2}. The examples and preferable ranges of the monovalent hetero ring group as the substituent which the amino group has are the same as the examples and preferable ranges of the monovalent hetero ring group represented by Ar^{H1} and Ar^{H2}.

At least one of Ar^{H1} and Ar^{H2} is preferably an aryl group or a monovalent hetero ring group, more preferably a monovalent hetero ring group, further preferably a carbazolyl group, a dibenzothienyl group or a dibenzofuryl group, and particularly preferably a carbazolyl group, since the light emitting device of the present embodiment is more excellent in light emission efficiency, and these groups optionally have a substituent.

Ar^{H1} and Ar^{H2} are each preferably an aryl group or a monovalent hetero ring group, more preferably a group obtained by removing from benzene, fluorene, pyridine, diazabenzene, triazine, carbazole, dibenzofuran or dibenzothiophene one hydrogen atom bonding directly to an atom constituting the ring, further preferably a phenyl group, a fluorenyl group, a dibenzothienyl group, a dibenzofuryl group or a carbazolyl group, and particularly preferably a carbazolyl group, since the light emitting device of the present embodiment is more excellent in light emission efficiency, and these groups optionally have a substituent.

At least one of L^{H1} is preferably an arylene group or a divalent hetero ring group, more preferably a divalent hetero ring group, and further preferably a group obtained by removing from carbazole, dibenzofuran or dibenzothiophene two hydrogen atoms bonding directly to atoms (preferably, carbon atoms) constituting the ring, since the light emitting device of the present embodiment is more excellent in light emission efficiency, and these groups optionally further have a substituent.

L^{H1} is preferably an arylene group or a divalent hetero ring group, more preferably a group obtained by removing from benzene, naphthalene, fluorene, pyridine, diazabenzene, triazine, azanaphthalene, diazanaphthalene, carbazole, dibenzofuran or dibenzothiophene two hydrogen atoms bonding directly to atoms (preferably, carbon atoms) constituting the ring, further preferably a group obtained by removing from benzene, fluorene, pyridine, diazabenzene, triazine, carbazole, dibenzofuran or dibenzothiophene two hydrogen atoms bonding directly to atoms (preferably, carbon atoms) constituting the ring, and particularly preferably a group obtained by removing from dibenzofuran or dibenzothiophene two hydrogen atoms bonding directly to atoms constituting the ring, since the light emitting device of the present embodiment is more excellent in light emission efficiency, and these groups optionally have a substituent.

The substituent which Ar^{H1}, Ar^{H2} and L^{H1} optionally have is preferably an alkyl group, a cycloalkyl group, an alkoxy group, a cycloalkoxy group, an aryl group, a monovalent hetero ring group, a substituted amino group or a fluorine atom, more preferably an alkyl group, a cycloalkyl group, an aryl group, a monovalent hetero ring group or a substituted amino group, further preferably an alkyl group, an aryl group or a monovalent hetero ring group, and these groups optionally further have a substituent.

The examples and preferable ranges of the aryl group, the monovalent hetero ring group and the substituted amino group as the substituent which Ar^{H1}, Ar^{H2} and L^{H1} optionally have are the same as the examples and preferable ranges of the aryl group, the monovalent hetero ring group and the substituted amino group represented by Ar^{H1} and Ar^{H2}, respectively.

The substituent which the substituent which Ar^{H1}, Ar^{H2} and L^{H1} optionally have optionally further has is preferably an alkyl group, a cycloalkyl group, an aryl group, a monovalent hetero ring group or a substituted amino group, more preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which Ar^{H1}, Ar^{H2} and L^{H1} optionally have optionally further has are the same as the examples and preferable ranges of the aryl group, the monovalent hetero ring group and the substituted amino group represented by Ar^{H1} and Ar^{H2}, respectively.

n^{H1} is usually an integer of 0 or more and 10 or less, preferably an integer of 0 or more and 5 or less, further preferably and integer of 1 or more and 3 or less, particularly preferably 1.

The compound represented by the formula (H-1) includes, for example, compounds represented by the following formulae. In the formulae, Z¹ represents an oxygen atom or a sulfur atom. In the formulae, Z² represents a group represented by -CH= or a group represented by -N=.

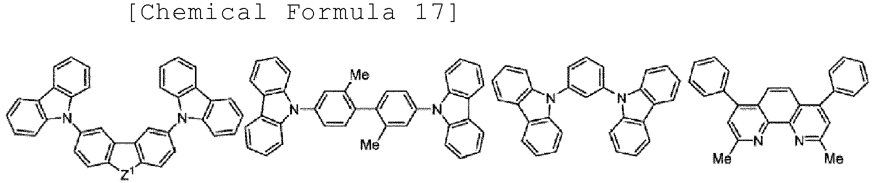

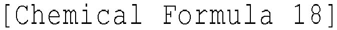

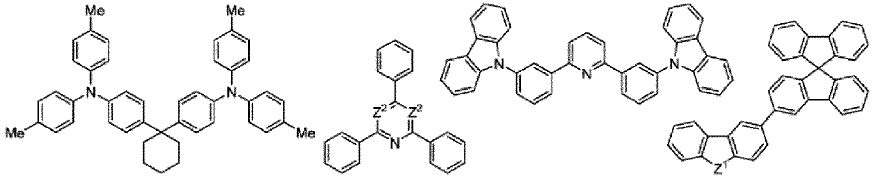

### [Other components]

The composition for light emitting device of the present embodiment may be a composition containing the metal complex represented by the formula (2) and the compound (B), and at least one selected from the group consisting of a host material, a hole transporting material, a hole injection material, an electron transporting material, an electron injection material, a light emitting material, an antioxidant and a solvent described above. The hole transporting material, the hole injection material, the electron transporting material, the electron injection material and the light emitting material are different from the metal complex represented by the formula (2) and the compound (B).

### [Ink]

The composition containing the metal complex represented by the formula (2) and the compound (B), and a solvent (hereinafter, referred to as "ink") is suitable for fabricating a light emitting device using a wet method such as, for example, a spin coat method, a casting method, a microgravure coat method, a gravure coat method, a bar coat method, a roll coat method, a wire bar coat method, a dip coat method, a spray coat method, a screen printing method, a flexo printing method, an offset printing method, an inkjet printing method, a capillary coat method, a nozzle coat method and the like. The viscosity of the ink may be adjusted according to the printing method, and is preferably 1 mPa•s to 20 mPa•s at 25°C.

The solvent contained in the ink is preferably a solvent capable of dissolving or uniformly dispersing solid components in the ink. The solvent includes, for example, chlorine-based solvents, ether-based solvents, aromatic hydrocarbon-based solvents, aliphatic hydrocarbon-based solvents, ketone-based solvents, ester-based solvents, polyhydric alcohol-based solvents, alcohol-based solvents, sulfoxide-based solvents and amide-based solvents.

In the ink, the compounding amount of the solvent is usually 1000 parts by mass to 10000000 parts by mass, when the sum of the metal complex represented by the formula (2) and the compound (B) is taken as 100 parts by mass.

The solvent may be used singly or in combination of two or more.

### • Hole transporting material

The hole transporting material is classified into low molecular weight compounds and polymer compounds, and polymer compounds having a cross-linkable group are preferable.

The polymer compound includes, for example, polyvinylcarbazole and derivatives thereof; and polyarylenes having an aromatic amine structure in the side chain or main chain, and derivatives thereof. The polymer compound may be a compound to which an electron accepting site such as fullerene, tetrafluorotetracyanoquinodimethane, tetracyanoethylene, trinitrofluorenone and the like is bonded.

In the composition for light emitting device of the present embodiment, when a hole transporting material is contained, the compounding amount of the hole transporting material is usually 1 part by mass to 10000 parts by mass, when the sum of the metal complex represented by the formula (2) and the compound (B) is taken as 100 parts by mass.

The hole transporting material may be used singly or in combination of two or more.

### • Electron transporting material

The electron transporting material is classified into low molecular weight compounds and polymer compounds. The electron transporting material may have a cross-linkable group.

The low molecular weight compound includes, for example, a metal complex having 8-hydroxyquinoline as a ligand, oxadiazole, anthraquinodimethane, benzoquinone, naphthoquinone, anthraquinone, tetracyanoanthraquinodimethane, fluorenone, diphenyldicyanoethylene and diphenoquinone, and derivatives thereof.

The polymer compound includes, for example, polyphenylene, polyfluorene, and derivatives thereof. The polymer compound may be doped with a metal.

In the composition for light emitting device of the present embodiment, when an electron transporting material is contained, the compounding amount of the electron transporting material is usually 1 part by mass to 10000 parts by mass, when the sum of the metal complex represented by the formula (2) and the compound (B) is taken as 100 parts by mass.

The electron transporting material may be used singly or in combination of two or more.

### • Hole injection material and electron injection material

The hole injection material and the electron injection material are each classified into low molecular weight compounds and polymer compounds. The hole injection material and the electron injection material may have a cross-linkable group.

The low molecular weight compound includes, for example, metal phthalocyanines such as copper phthalocyanine and the like; carbon; oxides of metals such as molybdenum, tungsten and the like; metal fluorides such as lithium fluoride, sodium fluoride, cesium fluoride, potassium fluoride and the like.

The polymer compound includes electrically conductive polymers such as, for example, polyaniline, polythiophene, polypyrrole, polyphenylenevinylene, polythienylenevinylene, polyquinoline and polyquinoxaline, and derivatives thereof; a polymer containing an aromatic amine structure in the main chain or side chain, and the like.

In the composition for light emitting device of the present embodiment, when a hole injection material and/or an electron injection material is contained, the compounding amounts of the hole injection material and the electron injection material are each usually 1 part by mass to 10000 parts by mass, when the sum of the metal complex represented by the formula (2) and the compound (B) is taken as 100 parts by mass.

The hole injection material and the electron injection material each may be used singly or in combination of two or more.

### • Ion doping

When the hole injection material or the electron injection material contains an electrically conductive polymer, the electric conductivity of the electrically conductive polymer is preferably 1×10⁻⁵ S/cm to 1×10³ S/cm. For adjusting the electric conductivity of the electrically conductive polymer within such a range, the electrically conductive polymer can be doped with an appropriate amount of ions. The kind of the ion to be doped is an anion for the hole injection material and a cation for the electron injection material. The anion includes, for example, a polystyrenesulfonic ion, an alkylbenzenesulfonic ion and a camphorsulfonic ion. The cation includes, for example, a lithium ion, a sodium ion, a potassium ion and a tetrabutylammonium ion.

The ions to be doped may be used singly or in combination of two or more.

### • Light emitting material

The light emitting material is classified into low molecular weight compounds and polymer compounds. The light emitting material may have a cross-linkable group.

The low molecular weight compound includes, for example, naphthalene and derivatives thereof, anthracene and derivatives thereof, perylene and derivatives thereof, and triplet light emitting complexes having iridium, platinum or europium as the central metal.

The triplet light emitting complex includes, for example, metal complexes shown below.

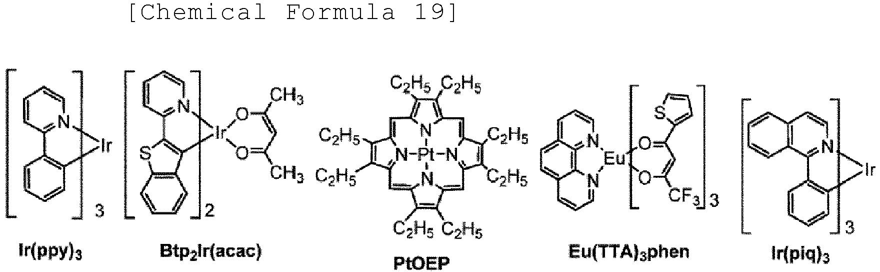

The polymer compound includes polymer compounds containing, for example, an arylene group such as a phenylene group, a naphthalenediyl group, a fluorenediyl group, a phenanthrenediyl group, a dihydrophenanthrenediyl group, an anthracenediyl group, a pyrenediyl group and the like; an aromatic amine residue such as a group obtained by removing from an aromatic amine two hydrogen atoms, and the like; and a divalent hetero ring group such as a carbazolediyl group, a phenoxazinediyl group, a phenothiazinediyl group and the like.

In the composition for light emitting device of the present embodiment, when a light emitting material is contained, the content of the light emitting material is usually 1 part by mass to 10000 parts by mass, when the sum of the metal complex represented by the formula (2) and the compound (B) is taken as 100 parts by mass.

The light emitting material may be used singly or in combination of two or more.

### • Antioxidant

The antioxidant may a compound which is soluble in the same solvent as for the metal complex represented by the formula (2) and the compound (B) and does not inhibit light emission and charge transportation, and includes, for example, phenol type antioxidants and phosphorus-based antioxidants.

In the composition for light emitting device of the present embodiment, when an antioxidant is contained, the compounding amount of the antioxidant is usually 0.00001 parts by mass to 10 parts by mass, when the sum of the metal complex represented by the formula (2) and the compound (B) is taken as 100 parts by mass.

The antioxidant may be used singly or in combination of two or more.

### <Film>

The film of the present embodiment contains the composition for light emitting device described above. The film of the present embodiment is suitable as a light emitting layer in a light emitting device. The film of the present embodiment can be fabricated, for example, by a wet method using an ink. Further, the film of the present embodiment can be fabricated, for example, by a dry method such as a vacuum vapor deposition method and the like. The method for fabricating the film of the present embodiment by a dry method includes, for example, a method of vapor-depositing the above-described composition for light emitting device and a method of co-vapor-depositing the metal complex represented by the formula (2) and the compound (B).

The thickness of the film is usually 1 nm to 10 µm.

### <Light emitting device>

The light emitting device of the present embodiment contains the composition for light emitting device described above.

The light emitting device of the present embodiment may be one having, for example, an anode, a cathode, and an organic layer containing the above-described composition for light emitting device disposed between the anode and the cathode.

### [Layer constitution]

The layer containing the composition for light emitting device of the present embodiment is usually one or more layers selected from the group consisting of a light emitting layer, a hole transporting layer, a hole injection layer, an electron transporting layer and an electron injection layer, and preferably is a light emitting layer. These layers contain a light emitting material, a hole transporting material, a hole injection material, an electron transporting material and an electron injection material, respectively. These layers can be formed by the same method as for the fabrication of the film described above using a light emitting material, a hole transporting material, a hole injection material, an electron transporting material and an electron injection material, respectively.

The light emitting device has a light emitting layer between an anode and a cathode. The light emitting device of the present embodiment preferably has at least one of a hole injection layer and a hole transporting layer between an anode and a light emitting layer, from the standpoint of hole injectability and hole transportability, and preferably has at least one of an electron injection layer and an electron transporting layer between a cathode and a light emitting layer, from the standpoint of electron injectability and electron transportability.

The materials of a hole transporting layer, an electron transporting layer, a light emitting layer, a hole injection layer and an electron injection layer include the hole transporting material, the electron transporting material, the light emitting material, the hole injection material and the electron injection material and the like described above, respectively, in addition to the composition for light emitting device of the present embodiment.

When the material of a hole transporting layer, the material of an electron transporting layer and the material of a light emitting layer are soluble in a solvent used in forming layers adjacent to the hole transporting layer, the electron transporting layer and the light emitting layer, respectively, in fabricating a light emitting device, it is preferable that the material has a cross-linkable group for avoiding the material from being dissolved in the solvent. After forming each layer using the material having a cross-linkable group, the cross-linkable group can be crosslinked to insolubilize the layer.

The method for forming each layer such as a light emitting layer, a hole transporting layer, an electron transporting layer, a hole injection layer, an electron injection layer and the like in the light emitting device of the present invention includes, for example, dry methods such as a method of vacuum vapor-deposition from a powder and the like and wet methods such as a method by film formation from a solution or melted state and the like when a low molecular weight compound is used, and includes, for example, wet methods such as a method by film formation from a solution or melted state and the like when a polymer compound is used. The order, number and thickness of layers to be laminated are adjusted in consideration of light emission efficiency, driving voltage and luminance life.

### [Substrate/electrode]

The substrate of a light emitting device may be a substrate on which an electrode can be formed and which does not chemically change in forming an organic layer, and it is, for example, a substrate made of a material such as glass, plastic, silicon and the like. In the case of an opaque substrate, it is preferable that the electrode farthest from the substrate is transparent or semi-transparent.

The material of the anode includes, for example, electrically conductive metal oxides and semi-transparent metals, preferably includes indium oxide, zinc oxide, tin oxide; electrically conductive compounds such as indium-tin-oxide (ITO), indium-zinc-oxide and the like; argentine-palladium-copper (APC) complex; NESA, gold, platinum, silver and copper.

The material of the cathode includes, for example, metals such as lithium, sodium, potassium, rubidium, cesium, beryllium, magnesium, calcium, strontium, barium, aluminum, zinc, indium and the like; alloys composed of two or more of them; alloys composed of one or more of them and one or more of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy and a calcium-aluminum alloy.

The anode and the cathode each may take a laminated structure composed of two or more layers.

### [Application]

The light emitting device of the present embodiment can be suitably used as a light source for backlight of a liquid crystal display device, a light source for illumination, organic EL illumination, a display device of computers, televisions, portable terminals and the like (for example, organic EL display and organic EL television).

Suitable embodiments of the present invention have been explained above, but the present invention is not limited to them.

### EXAMPLES

The present invention will be illustrated in detail by examples below, but the present invention is not limited to these examples.

In examples, the polystyrene-equivalent number-average molecular weight (Mn) and the polystyrene-equivalent weight-average molecular weight (Mw) of a polymer compound were determined by size exclusion chromatography (SEC) described below using tetrahydrofuran as the mobile phase.

A polymer compound to be measured was dissolved at a concentration of about 0.05% by mass in tetrahydrofuran, and 10 µL of the solution was injected into SEC. The mobile phase was flowed at a flow rate of 1.0 mL/min. As the column, PLgel MIXED-B (manufactured by Polymer Laboratories Ltd.) was used. As the detector, a UV-VIS detector (manufactured by Tosoh Corp., trade name: UV-8320GPC) was used.

For calculation of the value of ΔE_{ST} of a compound in examples, the ground state of the compound was structurally optimized by density-functional approach at B3LYP level, and in this procedure, 6-31G* was used as the basis function. Using Gaussian09 as the quantum chemistry calculation program, ΔE_{ST} of the compound was calculated by time-dependent density-functional approach at B3LYP level.

In examples, the maximum peak wavelength of the light emission spectrum of the metal complex was measured by a spectrophotometer (manufactured by JASCO Corporation, FP-6500) at room temperature. A metal complex was dissolved in xylene at a concentration of about 0.8×10⁻⁴% by mass and the resultant xylene solution was used as a specimen. As the excitation light, UV light having a wavelength of 325 nm was used.

### <Synthesis Example M> Synthesis of compounds M1 to M7

A compound M1 was synthesized according to a method described in International Publication WO2015/145871.

A compound M2 was synthesized according to a method described in International Publication WO2013/146806.

A compound M3 was synthesized according to a method described in International Publication WO2005/049546.

A compound M4 was synthesized according to a method described in JP-A No. 2010-189630.

A compound M5 and a compound M7 were synthesized according to a method described in International Publication WO2002/045184.

A compound M6 was synthesized according to a method described in International Publication WO2011/049241.

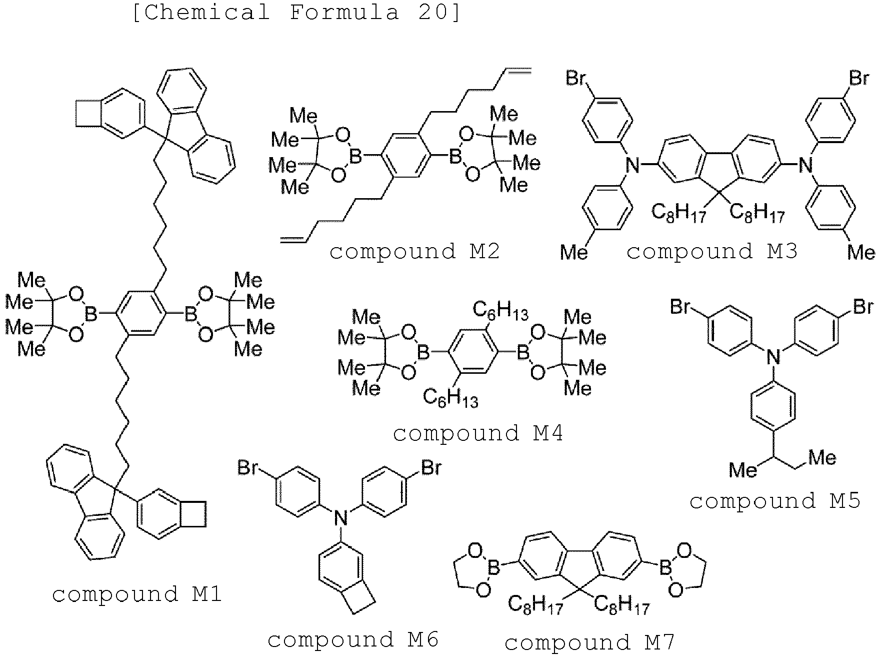

### <Synthesis Example HTL-1> Synthesis of polymer

### compound HTL-1

A polymer compound HTL-1 was synthesized according to a method described in International Publication WO2015/145871, using the compound M1, the compound M2 and the compound M3. The polymer compound HTL-1 had an Mn of 2.3×10⁴ and an Mw of 1.2×10⁵.

The polymer compound HTL-1 is a copolymer having a constitutional unit derived from the compound M1, a constitutional unit derived from the compound M2 and a constitutional unit derived from the compound M3 at a molar ratio of 45:5:50, according to the theoretical values calculated from the amounts of the charged raw materials.

### <Synthesis Example HTL-C1> Synthesis of polymer compound HTL-C1

A polymer compound HTL-C1 was synthesized according to a method described in International Publication WO2015/194448, using the compound M4 and the compound M3. The polymer compound HTL-C1 had an Mn of 4.5×10⁴ and an Mw of 1.5×10⁵.

The polymer compound HTL-C1 is a copolymer having a constitutional unit derived from the compound M4 and a constitutional unit derived from the compound M3 at a molar ratio of 50:50, according to the theoretical values calculated from the amounts of the charged raw materials.

### <Synthesis Example HTL-2> Synthesis of polymer compound HTL-2

A polymer compound HTL-2 was synthesized according to a method described in International Publication WO2011/049241, using the compound M7, the compound M5 and the compound M6. The polymer compound HTL-2 had an Mn of 8.9×10⁴ and an Mw of 4.2×10⁵.

The polymer compound HTL-2 is a copolymer having a constitutional unit derived from the compound M7, a constitutional unit derived from the compound M5 and a constitutional unit derived from the compound M6 at a molar ratio of 50:42.5:7.5, according to the theoretical values calculated from the amounts of the charged raw materials.

### <Synthesis and acquisition of metal complexes G1 to G4, Firpic and metal complexes R1 to R7>

A metal complex G1, a metal complex R2, a metal complex R3 and a metal complex R4 manufactured by Luminescence Technology corp. were used.

A metal complex G2 was synthesized according to a method described in JP-A No. 2013-237789.

A metal complex G3 was synthesized with reference to a method described in International Publication WO2009/131255.

A metal complex G4 was synthesized with reference to a method described in JP-A No. 2014-224101 and International Publication WO2009/131255.

Firpic manufactured by Aldrich was used.

A metal complex R1 manufactured by American Dye Source, Inc. was used.

A metal complex R5 was synthesized with reference to a method described in JP-A No. 2006-188673.

A metal complex R6 was synthesized according to a method described in JP-A No. 2011-105701.

A metal complex R7 was synthesized according to a method described in JP-A No. 2008-179617.

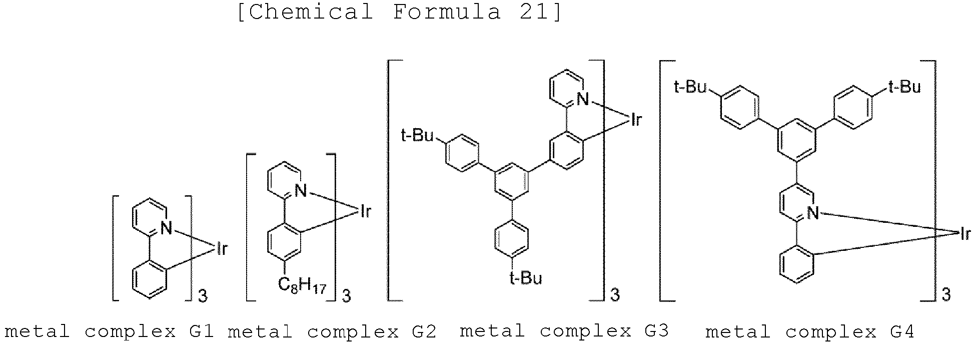

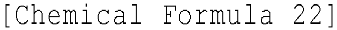

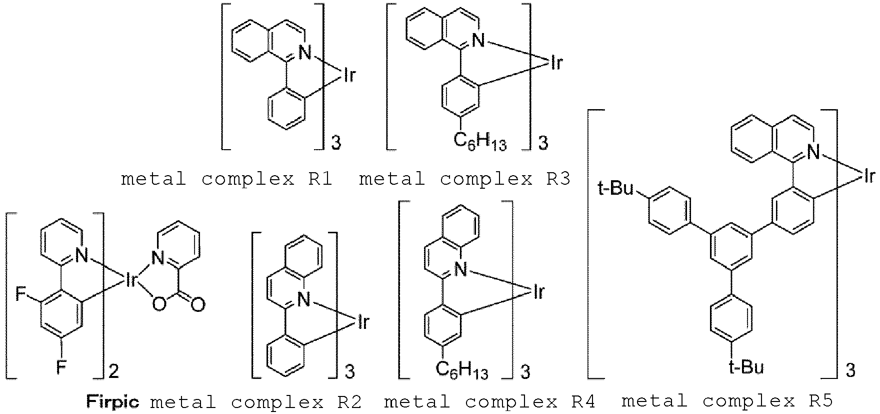

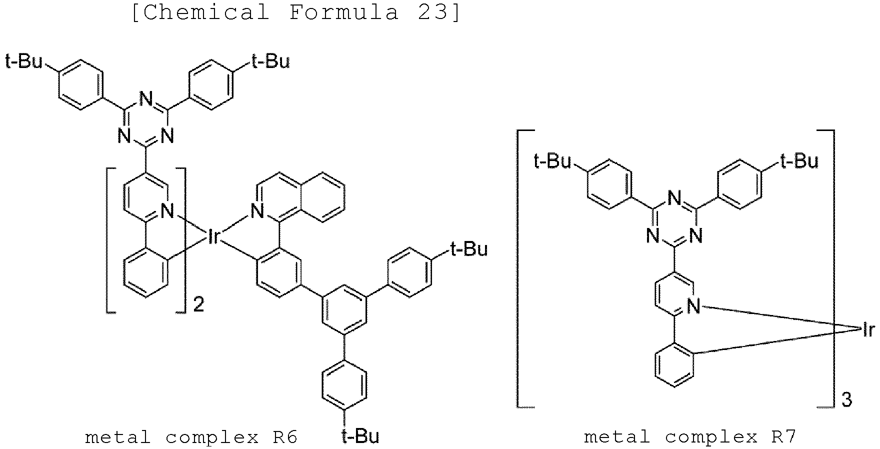

The maximum peak wavelength of the light emission spectrum of the metal complex G1 was 510 nm.

The maximum peak wavelength of the light emission spectrum of the metal complex G2 was 508 nm.

The maximum peak wavelength of the light emission spectrum of the metal complex G3 was 514 nm.

The maximum peak wavelength of the light emission spectrum of the metal complex G4 was 544 nm.

The maximum peak wavelength of the light emission spectrum of the Firpic was 470 nm.

The maximum peak wavelength of the light emission spectrum of the metal complex R1 was 618 nm.

The maximum peak wavelength of the light emission spectrum of the metal complex R2 was 579 nm.

The maximum peak wavelength of the light emission spectrum of the metal complex R3 was 620 nm.

The maximum peak wavelength of the light emission spectrum of the metal complex R4 was 580 nm.

The maximum peak wavelength of the light emission spectrum of the metal complex R5 was 619 nm.

The maximum peak wavelength of the light emission spectrum of the metal complex R6 was 611 nm.

The maximum peak wavelength of the light emission spectrum of the metal complex R7 was 594 nm.

### <Acquisition and synthesis of compounds H1, T1 and B1 to B3>

A compound H1 and a compound B1 manufactured by Luminescence Technology corp. were used.

A compound T1 was synthesized according to a method described in International Publication WO2018/062278.

A compound B2 and a compound B3 were synthesized with reference to a method described in International Publication WO2015/102118.

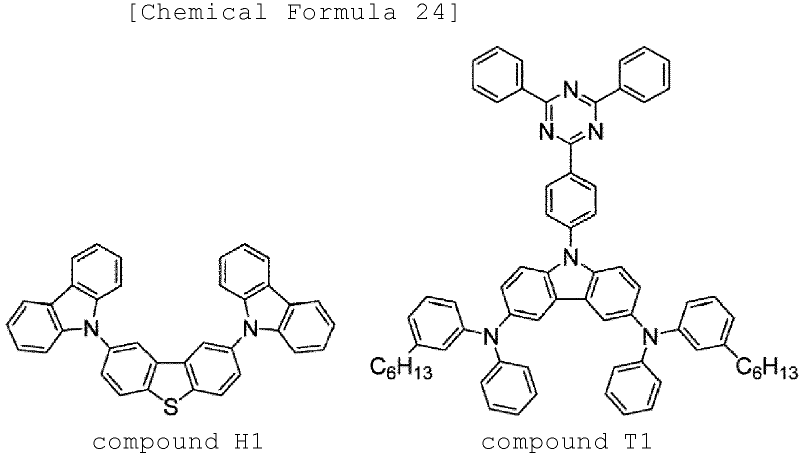

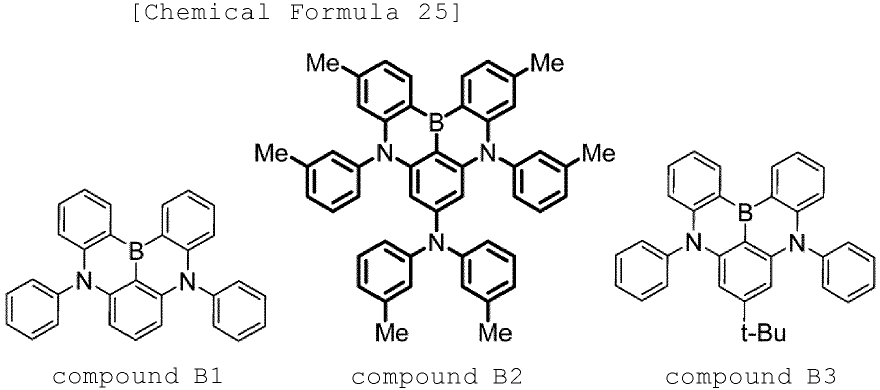

ΔEST of the compound T1 was 0.109 eV.
ΔEST of the compound B1 was 0.494 eV.
ΔEST of the compound B2 was 0.471 eV.
ΔEST of the compound B3 was 0.479 eV.

### <Example D1> Fabrication and evaluation of light emitting device D1

### (Formation of anode and hole injection layer)

An ITO film was attached with a thickness of 45 nm to a glass substrate by a sputtering method, to form an anode. On the anode, a hole injection material ND-3202 (manufactured by Nissan Chemical Industries, Ltd.) was spin-coated, to form a film with a thickness of 35 nm. The substrate carrying the hole injection layer laminated thereon was heated on a hot plate at 50°C for 3 minutes under an air atmosphere, and further heated at 230°C for 15 minutes, to form a hole injection layer.

### (Formation of hole transporting layer)

The polymer compound HTL-1 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a hole transporting layer.

### (Formation of light emitting layer)

The compound H1, the compound B1 and the metal complex G3 (compound H1/compound B1/metal complex G3 = 66% by mass/4% by mass/30% by mass) were dissolved at a concentration of 2% by mass in toluene. The resultant toluene solution was spin-coated on the hole transporting layer, to form a film with a thickness of 60 nm, and the film was heated at 130°C for 10 minutes under a nitrogen gas atmosphere, to form a light emitting layer.

### (Formation of cathode)

The substrate carrying the light emitting layer formed thereon was place in a vapor deposition machine, and the inner pressure thereof was reduced to 1.0×10⁻⁴ Pa or less, then, as the cathode, sodium fluoride was vapor-deposited with a thickness of 4 nm on the light emitting layer, then, aluminum was vapor-deposited with a thickness of about 80 nm on the sodium fluoride layer. After vapor deposition, the substrate carrying the cathode formed thereon was sealed with a glass substrate, to fabricate a light emitting device D1.

### (Evaluation of light emitting device)

Voltage was applied to the light emitting device D1 to observe EL light emission. The light emission efficiency [lm/W] at 100 cd/m² and the CIE chromaticity coordinate were measured.

### <Examples D2 to D3 and Comparative Examples CD1 to CD2>

Fabrication and evaluation of light emitting devices D2, D3, CD1 and CD2

Light emitting devices D2, D3, CD1 and CD2 were fabricated in the same manner as in Example D1, except that materials described in Table 1 were used instead of "the compound H1, the compound B1 and the metal complex G3 (compound H1/compound B1/metal complex G3 = 66% by mass/4% by mass/30% by mass)" in (Formation of light emitting layer) of Example D1.

Voltage was applied to the light emitting devices D2, D3, CD1 and CD2 to observe EL light emission. The light emission efficiency [lm/W] at 100 cd/m² and the CIE chromaticity coordinate were measured.

The results of Examples D1 to D3 and Comparative Examples CD1 to CD2 are shown in Table 1. The relative values of the light emission efficiency of the light emitting devices D1 to D3 and CD2, when the light emission efficiency of the light emitting device CD1 is taken as 1.0, are shown.

**[Table 1]**

| | Light emitting device | Light emitting layer | | Light emission efficiency (relative value) | CIE chromaticity coordinate (x, y) |
|---|---|---|---|---|---|
| | | material | Composition ratio (% by mass) | | |
| Example D1 | D1 | H1/B1/G3 | 66/4/30 | 11.8 | (0.30, 0.63) |
| Example D2 | D2 | H1/B1/G2 | 66/4/30 | 4.0 | (0.29, 0.62) |
| Example D3 | D3 | H1/B1/G4 | 66/4/30 | 13.4 | (0.43, 0.55) |
| Comparative Example CD1 | CD1 | H1/B1/G1 | 66/4/30 | 1.0 | (0.33, 0.61) |
| Comparative Example CD2 | CD2 | H1/B1/Firpic | 66/4/30 | 0.2 | (0.15, 0.26) |

### <Examples D4 to D6 and Comparative Example CD3>

Fabrication and evaluation of light emitting devices D4 to D6 and CD3

Light emitting devices D4 to D6 and CD3 were fabricated in the same manner as in Example D1, except that materials described in Table 2 were used instead of "the compound H1, the compound B1 and the metal complex G3 (compound H1/compound B1/metal complex G3 = 66% by mass/4% by mass/30% by mass)" in (Formation of light emitting layer) of Example D1.

Voltage was applied to the light emitting devices D4 to D6 and CD3 to observe EL light emission. The light emission efficiency [lm/W] at 2000 cd/m² and the CIE chromaticity coordinate were measured.

The results of Examples D4 to D6 and Comparative Example CD3 are shown in Table 2. The relative values of the light emission efficiency of the light emitting devices D4 to D6, when the light emission efficiency of the light emitting device CD3 is taken as 1.0, are shown.

**[Table 2]**

| | Light emitting device | Light emitting layer | | Light emission efficiency (relative value) | CIE chromaticity coordinate (x, y) |
|---|---|---|---|---|---|
| | | material | Composition ratio (% by mass) | | |
| Example D4 | D4 | H1/B1/G3 | 66/4/30 | 2.2 | (0.31, 0.64) |
| Example D5 | D5 | H1/B2/G3 | 66/4/30 | 2.7 | (0.31, 0.64) |
| Example D6 | D6 | H1/B3/G3 | 66/4/30 | 9.4 | (0.30, 0.63) |
| Comparative Example CD3 | CD3 | H1/T1/G3 | 66/4/30 | 1.0 | (0.30, 0.64) |

### <Examples D7 to D8 and Comparative Example CD4>

Fabrication and evaluation of light emitting devices D7, D8 and CD4

Light emitting devices D7, D8 and CD4 were fabricated in the same manner as in Example D1, except that materials described in Table 3 were used instead of "the compound H1, the compound B1 and the metal complex G3 (compound H1/compound B1/metal complex G3 = 66% by mass/4% by mass/30% by mass)" in (Formation of light emitting layer) of Example D1, and further, "the polymer compound HTL-C1" was used instead of "the polymer compound HTL-1" in (Formation of hole transporting layer) of Example D1.

Voltage was applied to the light emitting devices D7, D8 and CD4 to observe EL light emission. The light emission efficiency [lm/W] at 10000 cd/m² and the CIE chromaticity coordinate were measured.

The results of Examples D7 to D8 and Comparative Example CD4 are shown in Table 3. The relative values of the light emission efficiency of the light emitting devices D7 and D8, when the light emission efficiency of the light emitting device CD4 is taken as 1.0, are shown.

**[Table 3]**

| | Light emitting device | Light emitting layer | | Light emission efficiency (relative value) | CIE chromaticity coordinate (x, y) |
|---|---|---|---|---|---|
| | | Material | Composition ratio (% by mass) | | |
| Example D7 | D7 | H1/B1/G3 | 66/4/30 | 5.2 | (0.30, 0.64) |
| Example D8 | D8 | H1/B2/G3 | 66/4/30 | 3.8 | (0.30, 0.64) |
| Comparative Example CD4 | CD4 | H1/T1/G3 | 66/4/30 | 1.0 | (0.30, 0.64) |

### <Example D9> Fabrication and evaluation of light emitting device D9

### (Formation of anode and hole injection layer)

An ITO film was attached with a thickness of 45 nm to a glass substrate by a sputtering method, to form an anode. On the anode, a hole injection material ND-3202 (manufactured by Nissan Chemical Industries, Ltd.) was spin-coated, to form a film with a thickness of 50 nm. The substrate carrying the hole injection layer laminated thereon was heated on a hot plate at 50°C for 3 minutes, and further heated at 230°C for 15 minutes, under an air atmosphere, to form a hole injection layer.

### (Formation of hole transporting layer)

The polymer compound HTL-2 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a hole transporting layer.

### (Formation of light emitting layer)

The compound H1, the compound B1 and the metal complex R7 (compound H1/compound B1/metal complex R7 = 81% by mass/4% by mass/15% by mass) were dissolved at a concentration of 2% by mass in toluene. The resultant toluene solution was spin-coated on the hole transporting layer, to form a film with a thickness of 60 nm, and the film was heated at 130°C for 10 minutes under a nitrogen gas atmosphere, to form a light emitting layer.

### (Formation of cathode)

The substrate carrying the light emitting layer formed thereon was placed in a vapor deposition machine and the internal pressure thereof was reduced to 1.0×10⁻⁴ Pa or less, then, as the cathode, sodium fluoride was vapor-deposited with a thickness of about 4 nm on the light emitting layer, then, aluminum was vapor-deposited with a thickness of about 80 nm on the sodium fluoride layer. After vapor deposition, the substrate carrying the cathode formed thereon was sealed with a glass substrate, to fabricate a light emitting device D9.

### (Evaluation of light emitting device)

Voltage was applied to the light emitting device D9 to observe EL light emission. The light emission efficiency [lm/W] at 1000 cd/m² and the CIE chromaticity coordinate were measured.

### <Examples D10 to D11 and Comparative Example CD5>

Fabrication and evaluation of light emitting devices D10, D11 and CD5

Light emitting devices D10, D11 and CD5 were fabricated in the same manner as in Example D9, except that materials described in Table 4 were used instead of "the compound H1, the compound B1 and the metal complex R7 (compound H1/compound B1/metal complex R7 = 81% by mass/4% by mass/15% by mass)" in (Formation of light emitting layer) of Example D9.

Voltage was applied to the light emitting devices D10, D11 and CD5 to observe EL light emission. The light emission efficiency [lm/W] at 1000 cd/m² and the CIE chromaticity coordinate were measured.

The results of Examples D9 to D11 and Comparative Example CD5 are shown in Table 4. The relative values of the light emission efficiency of the light emitting devices D9 to D11, when the light emission efficiency of the light emitting device CD5 is taken as 1.0, are shown.

**[Table 4]**

| | Light emitting device | Light emitting layer | | Light emission efficiency (relative value) | CIE chromaticity coordinate (x, y) |
|---|---|---|---|---|---|
| | | Material | Composition ratio (% by mass) | | |
| Example D9 | D9 | H1/B1/R7 | 81/4/15 | 61.4 | (0.62, 0.38) |
| Example D10 | D10 | H1/B1/R6 | 81/4/15 | 16.7 | (0.65, 0.35) |
| Example D11 | D11 | H1/B1/R5 | 81/4/15 | 8.7 | (0.67, 0.32) |
| Comparative Example CD5 | CD5 | H1/B1/R1 | 81/4/15 | 1.0 | (0.41, 0.25) |

### <Examples D12 to D13 and Comparative Example CD6>

Fabrication and evaluation of light emitting devices D12, D13 and CD6

Light emitting devices D12, D13 and CD6 were fabricated in the same manner as in Example D9, except that materials described in Table 5 were used instead of "the compound H1, the compound B1 and the metal complex R7 (compound H1/compound B1/metal complex R7 = 81% by mass/4% by mass/15% by mass)" in (Formation of light emitting layer) of Example D9.

Voltage was applied to the light emitting devices D12, D13 and CD6 to observe EL light emission. The light emission efficiency [lm/W] at 2500 cd/m² and the CIE chromaticity coordinate were measured.

The results of Examples D12 to D13 and Comparative Example CD6 are shown in Table 5. The relative values of the light emission efficiency of the light emitting devices D12 and D13, when the light emission efficiency of the light emitting device CD6 is taken as 1.0, are shown.

**[Table 5]**

| | Light emitting device | Light emitting layer | | Light emission efficiency (relative value) | CIE chromaticity coordinate (x, y) |
|---|---|---|---|---|---|
| | | Material | Composition ratio (% by mass) | | |
| Example D12 | D12 | H1/B1/R4 | 81/4/15 | 17.5 | (0.58, 0.42) |
| Example D13 | D13 | H1/B1/R3 | 81/4/15 | 3.4 | (0.67, 0.32) |
| Comparative Example CD6 | CD6 | H1/B1/R2 | 81/4/15 | 1.0 | (0.48, 0.38) |

### Industrial Applicability

The composition of the present invention is useful for production of a light emitting device excellent in light emission efficiency.

## Claims

1. A light emitting device comprising
an anode,
a cathode, and
an organic layer disposed between said anode and said cathode and containing a composition for light emitting device, wherein
said composition for light emitting device contains
a metal complex represented by the formula (2), and
a compound (B) having a condensed hetero ring skeleton (b) containing a boron atom and at least one selected from the group consisting of an oxygen atom, a sulfur atom, a selenium atom, an sp³ carbon atom and a nitrogen atom in the ring: 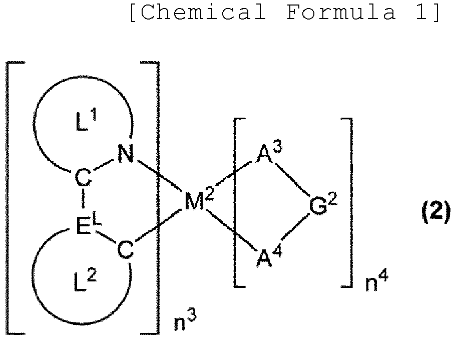 wherein,
M² represents a rhodium atom, a palladium atom, an iridium atom or a platinum atom,
n³ represents an integer of 1 or more, and n⁴ represents an integer of 0 or more, n³+n⁴ is 3 when M² is a rhodium atom or an iridium atom, while n³+n⁴ is 2 when M² is a palladium atom or a platinum atom,
E^{L} represents a carbon atom or a nitrogen atom, when a plurality of E^{L} are present, they may be the same or different,
Ring L¹ represents an aromatic hetero ring containing a 6-membered ring, and this ring optionally has a substituent, when a plurality of the substituents are present, they may be the same or different and may be combined together to form a ring together with atoms to which they are attached, when a plurality of Ring L¹ are present, they may be the same or different,
Ring L² represents an aromatic hydrocarbon ring or an aromatic hetero ring, and these rings optionally have a substituent, when a plurality of the substituents are present, they may be the same or different and may be combined together to form a ring together with atoms to which they are attached, when a plurality of Ring L² are present, they may be the same or different,
the substituent which Ring L¹ optionally has and the substituent which Ring L² optionally has may be the same or different and may be combined together to form a ring together with atoms to which they are attached,
at least one of Ring L¹ and Ring L² has a group represented by the formula (1-T) as the substituent, when a plurality of the groups represented by the formula (1-T) are present, they may be the same or different,
A³-G²-A⁴ represents an anionic bidentate ligand, A³ and A⁴ each independently represent a carbon atom, an oxygen atom or a nitrogen atom, and these atoms may be ring-constituent atoms, G² represents a single bond, or an atomic group constituting a bidentate ligand together with A³ and A⁴, when a plurality of A³-G²-A⁴ are present, they may be the same or different,
[Chemical Formula 2] **-R^{1T} (1-T)**
wherein, R^{1T} represents an alkyl group, a cycloalkyl group, an alkoxy group, a cycloalkoxy group, an aryloxy group, an aryl group, a monovalent hetero ring group or a substituted amino group, and these groups optionally have a substituent, when a plurality of the substituents are present, they may be the same or different and may be combined together to form a ring together with atoms to which they are attached.

2. The light emitting device according to Claim 1, wherein said Ring L¹ is a pyridine ring, a diazabenzene ring, an azanaphthalene ring or a diazanaphthalene ring, and these rings optionally have a substituent.

3. The light emitting device according to Claim 2, wherein
said Ring L¹ is a pyridine ring, a diazabenzene ring, a quinoline ring or a diazanaphthalene ring, and these rings optionally have a substituent, and
said R^{1T} is an alkyl group, a cycloalkyl group, an aryl group, a monovalent hetero ring group or a substituted amino group, and these groups optionally have a substituent.

4. The light emitting device acording to Claim 2, wherein
said Ring L¹ is an isoquinoline ring optionally having a substituent, and
said R^{1T} is an aryl group, a monovalent hetero ring group or a substituted amino group, and these groups optionally have a substituent.

5. The light emitting device according to any one of Claims 1 to 4, wherein
said Ring L² is a benzene ring, a pyridine ring or a diazabenzene ring, and these rings optionally have a substituent.

6. The light emitting device according to any one of Claims 1 to 5, wherein
said R^{1T} is an aryl group optionally having a substituent or a monovalent hetero ring group optionally having a substituent.

7. The light emitting device according to any one of Claims 1 to 6, wherein
said condensed hetero ring skeleton (b) contains a boron atom and at least one selected from the group consisting of an oxygen atom, a sulfur atom and a nitrogen atom in the ring.

8. The light emitting device according to Claim 7, wherein
said condensed hetero ring skeleton (b) contains a boron atom and a nitrogen atom in the ring.

9. The light emitting device according to any one of Claims 1 to 6, wherein
said compound (B) is a compound represented by the formula (1-1), a compound represented by the formula (1-2) or a compound represented by the formula (1-3): 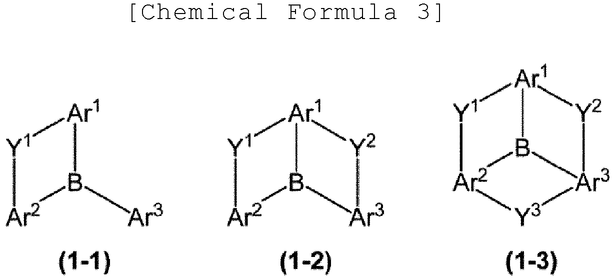 wherein,
Ar¹, Ar² and Ar³ each independently represent an aromatic hydrocarbon group or a hetero ring group, and these groups optionally have a substituent, when a plurality of the substituents are present, they may be the same or different and may be combined together to form a ring together with atoms to which they are attached,
Y¹ represents an oxygen atom, a sulfur atom, a selenium atom, a group represented by -N(Ry)-, an alkylene group or a cycloalkylene group, and these groups optionally have a substituent, when a plurality of the substituents are present, they may be the same or different and may be combined together to form a ring together with atoms to which they are attached,
Y² and Y³ each independently represent a single bond, an oxygen atom, a sulfur atom, a selenium atom, a group represented by -N(Ry)-, an alkylene group or a cycloalkylene group, and these groups optionally have a substituent, when a plurality of the substituents are present, they may be the same or different and may be combined together to form a ring together with atoms to which they are attached, Ry represents a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of Ry are present, they may be the same or different, Ry may be bonded directly or via a connecting group to Ar¹, Ar² or Ar³.

10. The light emitting device according to Claim 9, wherein
said Y¹, said Y² and said Y³ are each an oxygen atom, a sulfur atom or a group represented by -N(Ry)-.

11. The light emitting device according to Claim 10, wherein
said Y¹, said Y² and said Y³ are each a group represented by -N(Ry)-.

12. The light emitting device according to any one of Claims 1 to 11, wherein
the absolute value of a different between the energy level of the lowest triplet excited state of said compound (B) and the energy level of the lowest singlet excited state of said compound (B) is 0, 50 eV or less.

13. The light emitting device according to any one of Claims 1 to 12, wherein
said composition for light emitting device further contains a compound represented by the formula (H-1): 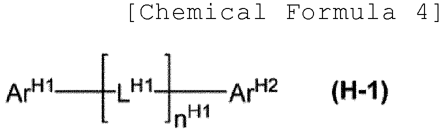 wherein,
Ar^{H1} and Ar^{H2} each independently represent an aryl group, a monovalent hetero ring group or a substituted amino group, and these groups optionally have a substituent, when a plurality of the substituents are present, they may be the same or different and may be combined together to form a ring together with atoms to which they are attached,
n^{H1} represents an integer of 0 or more,
L^{H1} represents an arylene group, a divalent hetero ring group, an alkylene group or a cycloalkylene group, and these groups optionally have a substituent, when a plurality of the substituents are present, they may be the same or different and may be combined together to form a ring together with atoms to which they are attached, when a plurality of L^{H1} are present, they may be the same or different.

14. The light emitting device according to any one of Claims 1 to 13, wherein
said composition for light emitting device further contains at least one selected from the group consisting of a hole transporting material, a hole injection material, an electron transporting material, an electron injection material, a light emitting material, an antioxidant and a solvent.

15. A composition for light emitting device comprising
a metal complex represented by the formula (2), and
a compound (B) having a condensed hetero ring skeleton (b) containing a boron atom and at least one selected from the group consisting of an oxygen atom, a sulfur atom, a selenium atom, an sp³ carbon atom and a nitrogen atom in the ring: 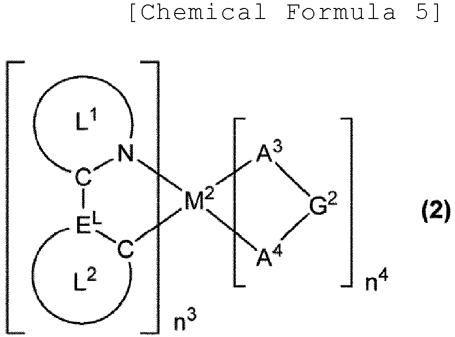 wherein,
M² represents a rhodium atom, a palladium atom, an iridium atom or a platinum atom,
n³ represents an integer of 1 or more, and n⁴ represents an integer of 0 or more, n³+n⁴ is 3 when M² is a rhodium atom or an iridium atom, while n³+n⁴ is 2 when M² is a palladium atom or a platinum atom,
E^{L} represents a carbon atom or a nitrogen atom, when a plurality of E^{L} are present, they may be the same or different,
Ring L¹ represents an aromatic hetero ring containing a 6-membered ring, and this ring optionally has a substituent, when a plurality of the substituents are present, they may be the same or different and may be combined together to form a ring together with atoms to which they are attached, when a plurality of Ring L¹ are present, they may be the same or different,
Ring L² represents an aromatic hydrocarbon ring or an aromatic hetero ring, and these rings optionally have a substituent, when a plurality of the substituents are present, they may be the same or different and may be combined together to form a ring together with atoms to which they are attached, when a plurality of Ring L² are present, they may be the same or different,
the substituent which Ring L¹ optionally has and the substituent which Ring L² optionally has may be the same or different and may be combined together to form a ring together with atoms to which they are attached,
at least one of Ring L¹ and Ring L² has a group represented by the formula (1-T) as the substituent, when a plurality of the groups represented by the formula (1-T) are present, they may be the same or different,
A³-G²-A⁴ represents an anionic bidentate ligand, A³ and A⁴ each independently represent a carbon atom, an oxygen atom or a nitrogen atom, and these atoms may be ring-constituent atoms, G² represents a single bond, or an atomic group constituting a bidentate ligand together with A³ and A⁴, when a plurality of A³-G²-A⁴ are present, they may be the same or different,
[Chemical Formula 6] **-R^{1T} (1-T)**
wherein, R^{1T} represents an alkyl group, a cycloalkyl group, an alkoxy group, a cycloalkoxy group, an aryloxy group, an aryl group, a monovalent hetero ring group or a substituted amino group, and these groups optionally have a substituent, when a plurality of the substituents are present, they may be the same or different and may be combined together to form a ring together with atoms to which they are attached.

16. The composition for light emitting device according to Claim 15, wherein
said Ring L¹ is a pyridine ring, a diazabenzene ring, a quinoline ring or a diazanaphthalene ring, and these rings optionally have a substituent, and
said R^{1T} is an alkyl group, a cycloalkyl group, an aryl group, a monovalent hetero ring group or a substituted amino group, and these groups optionally have a substituent.

17. The composition for light emitting device according to Claim 15, wherein
said Ring L¹ is an isoquinoline ring optionally having a substituent, and said R^{1T} is an aryl group, a monovalent hetero ring group or a substituted amino group, and these groups optionally have a substituent.

18. The composition for light emitting device according to any one of Claims 15 to 17, wherein
said condensed hetero ring skeleton (b) contains a boron atom and a nitrogen atom in the ring.

19. The composition for light emitting device according to any one of Claims 15 to 18,
further comprising a compound represented by the formula (H-1): 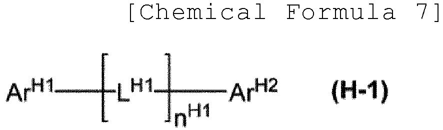 wherein,
Ar^{H1} and Ar^{H2} each independently represent an aryl group, a monovalent hetero ring group or a substituted amino group, and these groups optionally have a substituent, when a plurality of the substituents are present, they may be the same or different and may be combined together to form a ring together with atoms to which they are attached,
n^{H1} represents an integer of 0 or more,
L^{H1} represents an arylene group, a divalent hetero ring group, an alkylene group or a cycloalkylene group, and these groups optionally have a substituent, when a plurality of the substituents are present, they may be the same or different and may be combined together to form a ring together with atoms to which they are attached, when a plurality of L^{H1} are present, they may be the same or different.

20. The composition for light emitting device according to any one of Claims 15 to 19,
further comprising at least one selected from the group consisting of a hole transporting material, a hole injection material, an electron transporting material, an electron injection material, a light emitting material, an antioxidant and a solvent.
